# EUROPEAN PATENT SPECIFICATION

(11) **EP 2 972 363 B1**
(45) Date of publication and mention of the grant of the patent: **09.10.2019**
(21) Application number: 14782337.1
(22) Date of filing: 14.03.2014
(51) Int. Cl.: G01N 33/543, G01N 33/566, A61K 9/127, A61K 31/7048, C08G 83/00, A61K 47/59, A61K 47/62, A61K 47/64, A61P 37/04

(54) **IMMUNE-POTENTIATING DRUG NANOCARRIERS**
IMMUNPOTENZIERENDE WIRKSTOFFNANOTRÄGER
NANOVECTEURS POUR MÉDICAMENTS À EFFET DE POTENTIALISATION IMMUNITAIRE

(30) Priority: 15.03.2013 US 201361788666 P
(43) Date of publication of application: 20.01.2016
(73) Proprietor: University Of Miami, Miami, FL 33136 (US)
(72) Inventor: DAFTARIAN, Pirouz M., Palmetto Bay Florida 33157 (US); PEREZ, Victor, Pinecrest Florida 33156 (US); AGER, Arba LeRoy, Miami Florida 33186 (US)
(74) Representative: Gulde & Partner
(86) International application number: PCT/US2014/027457
(87) International publication number: WO 2014/168724

(56) References cited:
- WO-A1-2010/135394
- WO-A1-2013/034273
- WO-A1-2014/120804
- WO-A2-2010/115046
- US-A1- 2012 093 761
- US-A1- 2012 129 199
- LO-MAN RICHARD ET AL: "A fully synthetic therapeutic vaccine candidate targeting carcinoma-associated Tn carbohydrate antigen induces tumor-specific antibodies in nonhuman primates", CANCER RESEARCH, AMERICAN ASSOCIATION FOR CANCER RESEARCH, US, vol. 64, no. 14, 15 July 2004 (2004-07-15) , pages 4987-4994, XP002415006, ISSN: 0008-5472, DOI: 10.1158/0008-5472.CAN-04-0252
- WALSH, TS.: 'Safety, tolerance, and pharmacokinetics of a small unilamellar liposomal formulation of amphotericin B (AmBisome) in neutropenic patients.' ANTIMICROBIAL AGENTS AND CHEMOTHERAPY. vol. 42, 1998, pages 2391 - 2398, XP055283988
- GILEAD. AMBISOME (AMPHOTERICIN B) LIPOSOME FOR INJECTION. March 2012, page 1, XP055093404 Retrieved from the Internet: <URL:http://www.gilead.com/-/media/Files/pd fs/medicines/other/ambisome/ambisome_pi.pdf > [retrieved on 2014-06-20]
- AFRIN, F ET AL.: 'Characterization of Leishmania donovani antigens encapsulated in liposomes that induce protective immunity in BALB/c mice.' INFECTION AND IMMUNITY. vol. 70, no. 12, 2002, pages 6697 - 6706, XP055284343
- DAFTARIAN, PM ET AL.: 'A targeted and adjuvanted nanocarrier lowers the effective dose of liposomal amphotericin B and enhances adaptive immunity in murine cutaneous Leishmaniasis.' THE JOURNAL OF INFECTIOUS DISEASES. vol. 208, 2013, pages 1914 - 1922, XP055284512

## Description

### CROSS-REFERENCE TO RELATED APPLICATIONS

This application claims the benefit of Provisional Application Serial No. 61/788,666, filed March 15, 2013.

### SEQUENCE LISTING

The instant application contains a Sequence Listing which has been submitted electronically in ASCII format. Said ASCII copy, created on March 13, 2014, is named 7230-170WO_SL.txt and is 11,717 bytes in size.

### FIELD OF THE INVENTION

The invention, as defined by the claims, relates generally to the fields of chemistry, infectious diseases and immunology. More particularly, compositions, kits, platforms and methods for delivering drugs and other therapeutic agents to antigen presenting cells in a subject are disclosed herein.

### BACKGROUND

Unanticipated drug-induced toxicity is a major obstacle for drug development programs. Development of target/selective therapies (targeted drug delivery) is imperative, particularly when dealing with drugs possessing a broad range of toxicities. Leishmaniasis has become a global health concern in 88 countries having endemic status with about half a billion people being at risk. The majority of infections occur in Afghanistan, Iraq, Yemen, Saudi Arabia, Peru and Brazil. Unfortunately, traditional treatment strategies have failed to cure infections because of drug resistance and ineffective drugs. The poor drug uptake by mononuclear phagocytes, the parasite reservoir, and the associated toxicity of anti-Leishmania drugs has negatively affected their usefulness.

In WO 2010/135394 A1 compositions are disclosed for in vitro antigen presentation, assessing vaccine efficacy and assessing immunotoxicity of biologics and drugs. The compositions involve the combined use of MHC targeting, universal DR binding peptides with charged highly branched polymeric dendrimers. In WO 2010/115046 A2 and Lo-Man et al. ("A fully synthetic therapeutic vaccine candidate targeting carcinoma-associated Tn carbohydrate antigen induces tumor-specific antibodies in nonhuman primates", Cancer Research 2004, vol 64, pages 4987-4994) compositions have been disclosed for vaccine delivery and/or antibody generation, said compositions comprising a dendrimer conjugated with at least one MHC II targeting moiety and with at least one antigen.

### SUMMARY

The present invention is directed to subject matter as defined in the claims.

Any references in the description to methods of treatment refer to the compounds, pharmaceutical compositions and medicaments of the present invention for use in a method for treatment of the human or animal body by therapy.

Antigen presenting cells (APCs) including macrophages and dendritic cells are central in mounting protective immune responses against cancer and many parasites, bacteria, and viruses. They too dictate the tolerogenic immutherapies for autoimmune disorders, including diabetes. Thus, selective drug delivery to APCs has been the subject of much interest; in particular, when the pathogen can infect such cells and give rise to an outbreak of so many deadly and hard to treat diseases in humans including tuberculosis, human immunodeficiency virus (HIV), Rickettsial infections, and leishmaniasis. An APC targeting therapeutic delivery system will have major medicinal applications. We have created a peptide-dendrimer nanoparticle (PDD) that targets MHC class II molecules (expressed on macrophage, dendritic cells and other APCs) of human and mice indiscriminately. The platform is tailored to be complexed with payloads such as drug formulations including liposomes (for example liposome entrapped amphotericin B), biotherapeutics (for example albumin) or small molecules (dsRNA), to escort the payload onto APC, and to shuttle it into these cells. The platform has intrinsic immunopotentiating effects via its homing moiety, and a promiscuous CD4+ Th determinant that binds to murine, rats, monkeys and human MHC class II molecules expressed on their APCs.

Amphotericin-B is an effective anti-leishmaniasis agent but it induces significant nephrotoxicity. Complexing Amphotericin-B with liposomes (AmBisome®, also referred to herein as "AmB") reduces nephrotoxicity and improves AmB efficacy in treating leishmaniasis in humans. However, complicated dosing regimens are required to minimize side effects. Described herein are nanoparticle-based compositions, kits, methods and platforms for delivering anti-pathogen agents to APCs (including professional APCs (PAPCs)) *in vivo* that result in a robust and specific immune response to a pathogen, e.g., *Leishmania major.* The composition, kits, platforms and methods involve the combined use of MHC targeting and immunogenic peptides (e.g., PADRE, HA) with dendrimers (e.g., positively-charged highly branched polymeric dendrimers such as PAMAM (polyamidoamine) and other dendrimers) as vehicles for the targeted delivery of anti-pathogen agents (e.g., AmB) to PAPCs via MHC class II molecules expressed on the surface of such cells such that the anti-pathogen agent is internalized by the PAPCs. Typical compositions and nanocarriers described herein include a charged (e.g., positively-charged) polymeric dendrimer conjugated to an MHC targeting and immunogenic peptide such as a universal T helper peptide (e.g., an epitope such as the PADRE peptide or Influenza HA), and at least one therapeutic agent (e.g., a drug such as an anti-pathogen drug, an anti-pathogen drug coupled to a liposome, small molecule, etc.). The universal T helper peptide is conjugated to (e.g., covalently bound to) the exterior surface of the dendrimer such that the universal T helper peptide specifically binds to PAPCs, and the at least one drug is encapsulated by the dendrimer or conjugated to (e.g., covalently bound to) the exterior of the dendrimer. Such a composition or nanocarrier is capable of inducing an immune response against a pathogen (e.g., an intracellular pathogen such as *Leishmania*) when administered to a subject.

A typical nanocarrier system described herein has positively charged amine groups that make stable complexes with the negatively charged AmB and escorts the AmB directly into phagocytic cells *in vivo.* In one embodiment, charged (e.g., positively-charged) highly branched polymeric dendrimers conjugated to a T helper peptide (e.g., an epitope such as the PADRE peptide or Influenza HA) provide for a treatment of an intracellular pathogen with increased efficacy due to a reduced dose of anti-pathogen agent (e.g., AmB) compared to the dose(s) conventionally used. The composition, kits, platforms and methods described herein provide for specific and efficient delivery of anti-pathogen agents (e.g., drugs such as AmB) to PAPCs *in vivo* that results in a reduction of growth or elimination of the pathogen involving a robust and specific immune response to the pathogen. The nanocarriers described herein target phagocytes, shuttle their cargo into these cells, activate CD4 T helper cells, and act as an adjuvant to enhance immune responses.

Accordingly, described herein is a composition including at least one dendrimer having conjugated thereto at least one MHC II targeting moiety (e.g., a targeting peptide such as a universal T helper peptide) and at least one drug or other therapeutic agent, wherein the at least one MHC II targeting moiety is conjugated to the exterior surface of the at least one dendrimer such that the at least one MHC II targeting moiety specifically binds to APCs, and the at least one drug is encapsulated by the at least one dendrimer or conjugated to (e.g., covalently bound to) its exterior surface. The MHC II targeting moiety, e.g., targeting peptide, can be covalently attached to the at least one dendrimer. The at least one drug may include a liposome. In this embodiment, the liposome can be non-covalently attached to the at least one dendrimer. The at least one drug is an anti-pathogen agent, namely, the at least one drug is any form of a unilamellar liposomal formulation of amphotericin B. In one embodiment, the anti-pathogen agent is AmBisome® and is in an amount of about 0.1 mg/kg to about 50 mg/kg (e.g., for treating a *Leishmania* infection in a subject such as a mammal, the concentration of AmBisome® per dose is in the range of about 0.1 mg/kg to about 50 mg/kg). The composition may further include a pharmaceutically acceptable carrier. The at least one MHC II targeting moiety can be a Pan-DR T helper epitope (PADRE), and the at least one dendrimer can be a PAMAM dendrimer. The anti-pathogen agent is effective at reducing growth of or killing a pathogen such as, for example, *Leishmania,* malaria, tuberculosis, and human immunodeficiency virus (HIV). The at least one drug can be encapsulated in a liposome with a net-charge opposite to that of the at least one dendrimer (e.g., the charge of the at least one dendrimer is negative and the net-charge of the at least one drug and the liposome is positive, or the charge of the at least one dendrimer is positive and the net-charge of the at least one drug and the liposome is negative).

Also described herein is a method of delivering a drug or other therapeutic agent specifically to professional APCs in a subject (e.g., human) at risk of contracting or having an intracellular pathogen (e.g., *Leishmania,* malaria, tuberculosis, and HIV) infection. The method includes: administering to the subject an effective amount of a composition as described herein to induce MHC class II mediated activation of helper T cells in the subject, and to inhibit growth of or eliminate the intracellular pathogen, wherein administering the composition to the subject results in production of monoclonal antibodies against the intracellular pathogen. The at least one drug is an anti-pathogen agent, namely, any form of a unilamellar liposomal formulation of amphotericin B. The anti-pathogen agent can be AmBisome® and be present in an amount of about 0.1 mg/kg to about 50 mg/kg. The composition can further include a pharmaceutically acceptable carrier. The at least one MHC II targeting moiety (e.g., a targeting peptide such as a universal T helper peptide) can be PADRE, and the at least one dendrimer can be a PAMAM dendrimer. In the method, administration of the composition results in no local adverse reactions in the subject, but results in internalization of the drug or other therapeutic agent by the professional APCs. In some embodiments, the drug is AmBisome® and at least a plurality of the professional APCs are infected with *Leishmania*, wherein the MHC class II mediated activation of helper T cells is specific for *Leishmania.* Administration of the composition induces increased production of IFN-γ in splenocytes of the subject. In some embodiments, the subject has at least one lesion, and administration of the composition results in a reduction in size of the at least one lesion.

Further described herein is a kit for treating or preventing infection by an intracellular pathogen in a subject. The kit includes: a composition as described herein; at least one buffer; instructions for use; and packaging. The composition may be lyophilized, and the at least one buffer can be, for example, physiological saline, phosphate buffer saline, or OptiMem. The at least one drug or other therapeutic agent can be an anti-pathogen agent, e.g., any form of a unilamellar liposomal formulation of amphotericin B.

Unless otherwise defined, all technical terms used herein have the same meaning as commonly understood by one of ordinary skill in the art to which this invention belongs.

As used herein, a "nucleic acid" or a "nucleic acid molecule" means a chain of two or more nucleotides such as RNA (ribonucleic acid) and DNA (deoxyribonucleic acid), and chemically-modified nucleotides. A "purified" nucleic acid molecule is one that is substantially separated from other nucleic acid sequences in a cell or organism in which the nucleic acid naturally occurs (e.g., 30, 40, 50, 60, 70, 80, 90, 95, 96, 97, 98, 99, 100% free of contaminants). The terms include, e.g., a recombinant nucleic acid molecule incorporated into a vector, a plasmid, a virus, or a genome of a prokaryote or eukaryote. Examples of purified nucleic acids include cDNAs, fragments of genomic nucleic acids, nucleic acids produced polymerase chain reaction (PCR), nucleic acids formed by restriction enzyme treatment of genomic nucleic acids, recombinant nucleic acids, and chemically synthesized nucleic acid molecules. A "recombinant" nucleic acid molecule is one made by an artificial combination of two otherwise separated segments of sequence, e.g., by chemical synthesis or by the manipulation of isolated segments of nucleic acids by genetic engineering techniques.

When referring to an amino acid residue in a peptide, oligopeptide or protein, the terms "amino acid residue", "amino acid" and "residue" are used interchangably and, as used herein, mean an amino acid or amino acid mimetic joined covalently to at least one other amino acid or amino acid mimetic through an amide bond or amide bond mimetic.

As used herein, "protein" and "polypeptide" are used synonymously to mean any peptide-linked chain of amino acids, regardless of length or post-translational modification, e.g., glycosylation or phosphorylation.

When referring to a nucleic acid molecule, polypeptide, or infectious pathogen, the term "native" refers to a naturally-occurring (e.g., a wild-type (WT)) nucleic acid, polypeptide, or infectious pathogen.

As used herein, the term "antigen" or "immunogen" means a molecule that is specifically recognized and bound by an antibody.

When referring to an epitope (e.g., T helper epitope), by biological activity is meant the ability to bind an appropriate MHC molecule and, in the case of peptides useful for stimulating CTL responses, induce a T helper response and a CTL response against a target antigen or antigen mimetic.

The terms "specific binding" and "specifically binds" refer to that binding which occurs between such paired species as enzyme/substrate, receptor/agonist, antibody/antigen, etc., and which may be mediated by covalent or non-covalent interactions or a combination of covalent and non-covalent interactions. When the interaction of the two species produces a non-covalently bound complex, the binding which occurs is typically electrostatic, hydrogen-bonding, or the result of lipophilic interactions. Accordingly, "specific binding" occurs between a paired species where there is interaction between the two which produces a bound complex having the characteristics of an antibody/antigen or enzyme/substrate interaction. In particular, the specific binding is characterized by the binding of one member of a pair to a particular species and to no other species within the family of compounds to which the corresponding member of the binding member belongs.

As used herein, the terms "Pan-DR epitope," "Pan DR T helper epitope," "Pan-HLA-DR-binding epitope," "PADRE" and "PADRE peptides" mean a peptide of between about 4 and about 20 residues that is capable of binding at least about 7 of the 12 most common DR alleles (DR1, 2w2b, 2w2a, 3, 4w4, 4w14, 5, 7, 52a, 52b, 52c, and 53) with high affinity. "High affinity" is defined herein as binding with an IC₅₀% of less than 200 nm. For example, high affinity binding includes binding with an IC₅₀% of less than 3100 nM. For binding to Class II MHC, a binding affinity threshold of 1,000 nm is typical, and a binding affinity of less than 100nm is generally considered high affinity binding. Construction and use of PADRE peptides is described in detail in U.S. Patent No. 5,736,142 which is incorporated herein by reference.

A "T helper peptide" as used herein refers to a peptide recognized by the T cell receptor of T helper cells. For example, the PADRE peptides described herein are T helper peptides.

As used herein, the terms "MHC class II" and "MHC II" mean major histocompatibility complex class II. In humans, MHC class II are also called "HLA-DR."

By the terms "MHC II targeting peptide" and "MHC class II targeting peptide" is meant any peptide that binds to an MHC class II molecule or domain thereof.

As used herein, the term "dendrimer" means a charged (e.g., positively-charged, negatively-charged) substantially spherical or substantially linear polymer or macromolecule ranging from approximately 5nm to approximately 50 nm in size that can be coupled to a moiety able to bind to MHC II, e.g., an MHC II targeting peptide such as a universal T helper peptide, CLIP, etc. An example of a dendrimer is a charged, highly branched polymeric macromolecule with roughly spherical shape. Such a dendrimer can be, for example, a positively-charged, highly branched polymeric PAMAM dendrimer. In a specific embodiment, a dendrimer is a highly branched macromolecule spanning from a central core and containing a series of layers, structurally and synthetically distinct, which are usually referred to as 'generations'.

When referring to a dendrimer, by the phrase "highly branched" is meant a polymer with branched architecture with a high number of functional groups.

By the terms "PAMAM dendrimer" and "poly-amidoamine dendrimer" is meant a type of dendrimer in which tertiary amines are located at branching points and connections between structural layers are made by amide functional groups. PAMAM dendrimers exhibit many positive charges on their surfaces. PAMAM with many different surface groups, e.g., amidoethanol , midoethylethanolamine, amino, succinamic acid, hexlamide, etc., are commercially available.

By the term "derivatized dendrimer" is meant a dendrimer having one or more functional groups conjugated to its surface. A "PDD" is a peptide-derivatized dendrimer.

A "PADRE-derivatized dendrimer" or "PADRE-dendrimer" is a nanoconstruct (e.g., nanocarrier, nanovehicle) in which one or more PADRE peptides are covalently attached to the functional groups on the surface of a dendrimer (e.g., a PAMAM dendrimer).

As used herein, the term "professional antigen presenting cell" means cells that displays foreign antigens in the context of self major histocompatibility complex (MHC) on their surfaces and includes dendritic cells, macrophages, monocytes, and B cells.

By the term "conjugated" is meant when one molecule or agent is physically or chemically coupled or adhered to another molecule or agent. Examples of conjugation include covalent linkage (e.g., covalently bound drug or other small molecule) and electrostatic complexation. The terms "complexed," "complexed with," and "conjugated" are used interchangeably herein.

As used herein, the phrase "sequence identity" means the percentage of identical subunits at corresponding positions in two sequences (e.g., nucleic acid sequences, amino acid sequences) when the two sequences are aligned to maximize subunit matching, i.e., taking into account gaps and insertions. Sequence identity can be measured using sequence analysis software (e.g., Sequence Analysis Software Package from Accelrys CGC, San Diego, CA).

The phrases "isolated" or "biologically pure" refer to material which is substantially or essentially free from components which normally accompany it as found in its native state.

As used herein, the terms "nanoparticle," "nanovehicle" and "nanocarrier" mean a microscopic particle whose size is measured in nanometers. For example, a nanoparticle, nanovehicle or nanocarrier is a PADRE-dendrimer conjugate (e.g., PDD) or a particle combining several PADRE-dendrimer conjugates with a total diameter in the range of approximately 2-500 nm.

As used herein, the term "net-charge" means the sum of the electric charges of the particles or compounds (for example drugs, liposomes, or dendrimers) in a physiological pH.

As used herein, the term "therapeutic agent" is meant to encompass any molecule, chemical entity, composition, drug, or biological agent capable of preventing or reducing growth of a pathogen, or capable of blocking the ability of a pathogen to cause disease. An example of a therapeutic agent is a drug (e.g., any formulation type of Amphotericin B), including a drug conjugated to or encapsulated by a carrier such as a liposome. An example of such a drug is liposomal Amphotericin B, e.g., AmBisome®). The term "therapeutic agent" includes small molecules, antisense reagents, nucleic acids, siRNA reagents, antibodies, enzymes, polypeptides, peptides, organic or inorganic molecules, natural or synthetic compounds and the like.

The term "antibody" is meant to include polyclonal antibodies, monoclonal antibodies (mAbs), chimeric antibodies, humanized antibodies, anti-idiotypic (anti-Id) antibodies to antibodies that can be labeled in soluble or bound form, as well as fragments, regions or derivatives thereof, provided by any known technique, such as, but not limited to, enzymatic cleavage, peptide synthesis or recombinant techniques.

As used herein the term "adjuvant" means any material or substance which enhances the humoral and/or cellular immune response.

As used herein, the terms "displayed" or "surface exposed" are considered to be synonyms, and refer to antigens or other molecules that are present (e.g., accessible to immune site recognition) at the external surface of a structure such as a nanoparticle or nanocarrier (e.g., PADRE-dendrimer, HA-dendrimer, etc.).

The expression "biologically compatible form suitable for administration *in vivo*" as used herein means a form of the substance to be administered in which any toxic effects are outweighed by the therapeutic effects. The substances may be administered to any subject, e.g., humans.

By the phrase "immune response" is meant induction of antibody and/or immune cell-mediated responses specific against an antigen, antigens, pathogen, pathogenic agent, etc. An immune response has many facets, some of which are exhibited by the cells of the immune system (e.g., B-lymphocytes, T-lymphocytes, macrophages, and plasma cells). Immune system cells may participate in the immune response through interaction with an antigen or pathogen or other cells of the immune system, the release of cytokines and reactivity to those cytokines. Immune responses are generally divided into two main categories--humoral and cell-mediated. The humoral component of the immune response includes production of antibodies specific for an antigen or pathogen. The cell-mediated component includes the generation of delayed-type hypersensitivity and cytotoxic effector cells against the antigen or pathogen. An immune response can include, for example, activation of a CD4 T helper response.

By the phrases "therapeutically effective amount" and "effective dosage" is meant an amount sufficient to produce a therapeutically (e.g., clinically) desirable result; the exact nature of the result will vary depending on the nature of the disorder being treated. For example, where the disorder to be treated is a pathogenic infection, the result can be elimination of the pathogen, a reduction in growth of the pathogen, a reduction in size or elimination of a lesion associated with the pathogen, etc. The compositions and nanocarriers described herein can be administered from one or more times per day to one or more times per week. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of the compositions and nanocarriers described herein can include a single treatment or a series of treatments.

As used herein, the term "treatment" is defined as the application or administration of a therapeutic agent described herein, or identified by a method described herein, to a patient, or application or administration of the therapeutic agent to an isolated tissue or cell line from a patient, who has a disease, a symptom of disease or a predisposition toward a disease, with the purpose to cure, heal, alleviate, relieve, alter, remedy, ameliorate, improve or affect the disease, the symptoms of disease, or the predisposition toward disease.

The terms "patient" "subject" and "individual" are used interchangeably herein, and mean an animal to be treated, including vertebrates and invertebrates. Typically, a subject is a human. In some cases, the methods described herein find use in experimental animals, in veterinary applications (e.g., equine, bovine, ovine, canine, feline, avian, etc.), and in the development of animal models for disease, including, but not limited to, rodents including mice, rats, and hamsters, as well as non-human primates.

Although compositions, kits, platforms and methods similar or equivalent to those described herein can be used in the practice or testing of the present invention, suitable compositions, kits, platforms and methods are described below. In the case of conflict, the present specification, including definitions, will control. The particular embodiments discussed below are illustrative only and not intended to be limiting.

### BRIEF DESCRIPTION OF THE DRAWINGS

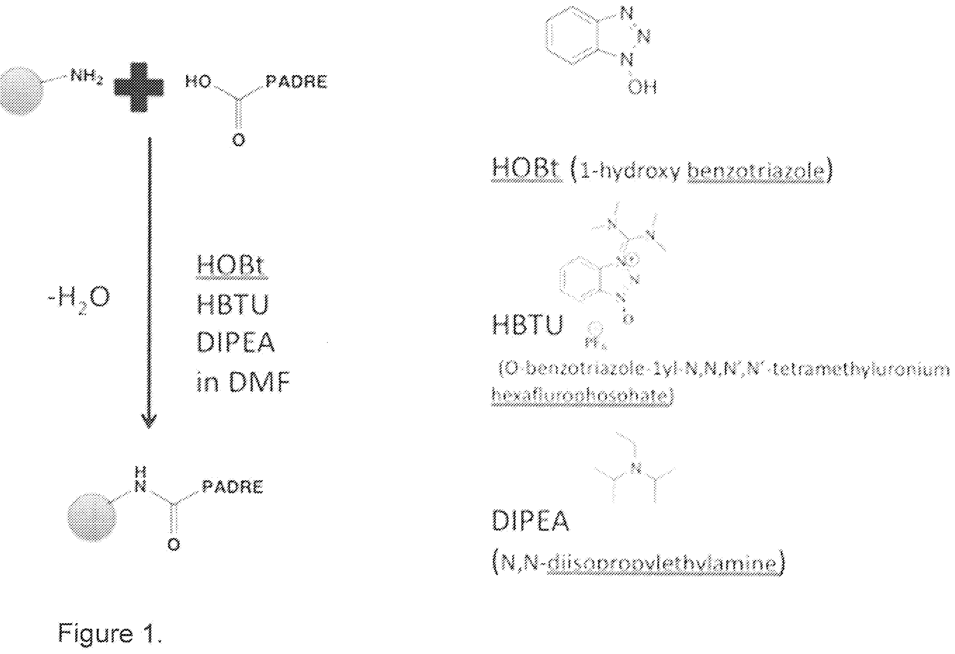

FIG. 1 is a schematic demonstrating the synthesis of PADRE-dendrimer. They are bonded together using an amide coupling reaction. On average, according to mass spectrometry, about two MHC class II homing peptides are attached per dendrimer. Once the nanoparticle is made it can be complexed with a therapeutic agent such as biotherapeutic, a drug (e.g., liposomal amphotericin B or AmBisome®), small molecule, etc. After this complex is made and injected, the APC homing marker will find the MHC class II receptor and bind to it.

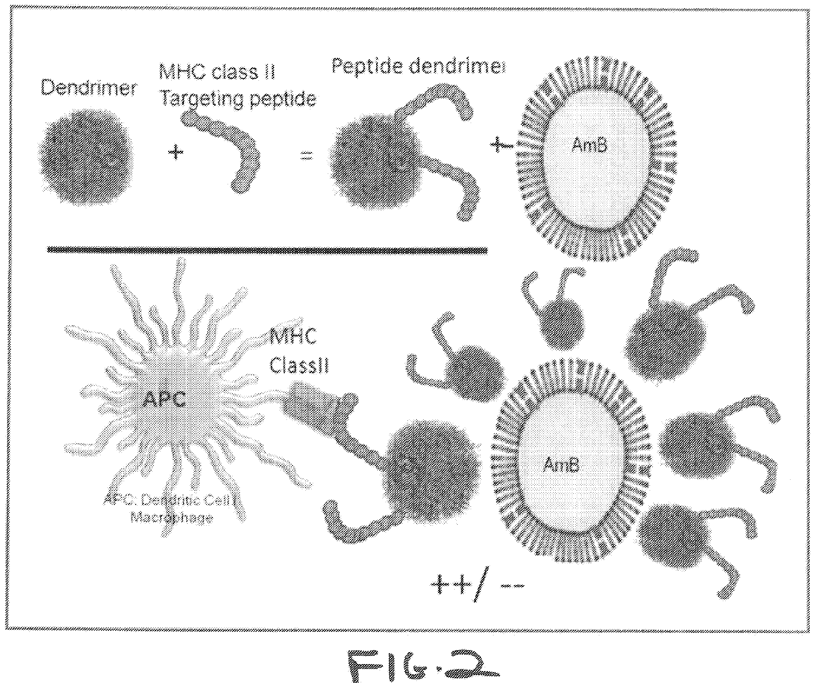

FIG. 2 is a schematic illustrating a nanoparticle to home drugs to mononuclear phagocytes. Macrophages express MHC class II and are the reservoir for *Leishmania* parasites. This figure depicts a drug-carrying nanocarrier platform that targets cells expressing MHC class II. Macrophage, monocytes, and DCs are the reservoir for *Leishmania* parasites and express MHC class II, a molecule that PDD nanocarriers as described herein target. Peptide-dendrimers are a platform for homing AmB cells expressing MHC class II (PAPCs). Peptides with high affinity bind specifically to macrophages and are covalently linked to the dendrimer; the platform is positively charged and makes a strong complex with negatively-charged AmB targeting it to the MHC class II⁺ phagocytes/dendritic cells (DCs). A homing peptide is a universal T helper epitope that binds to the flank of MHC class II expressed in all APCs.

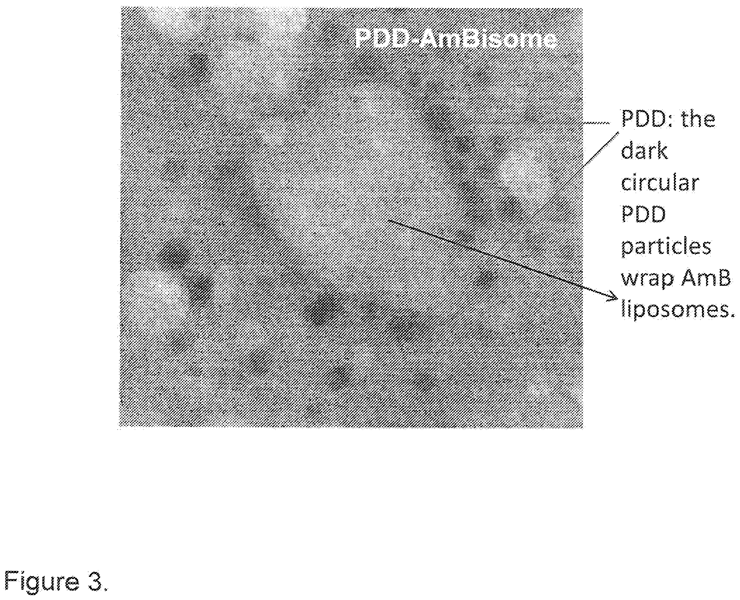

FIG. 3 is a TEM section showing PDD particles making complex and covering AmB. AmB was mixed with PDD at a weight ratio of 1:10. The mixture was incubated at room temperature for 20 minutes to form. A drop of the sample was placed on a carbon and nitrocellulose coated 200 mesh copper grid and allowed to air dry. The grids were then examined at several magnifications up to 245,000X in a Philips CM-10 TEM. Images were taken of each preparation using a Gatan digital camera.

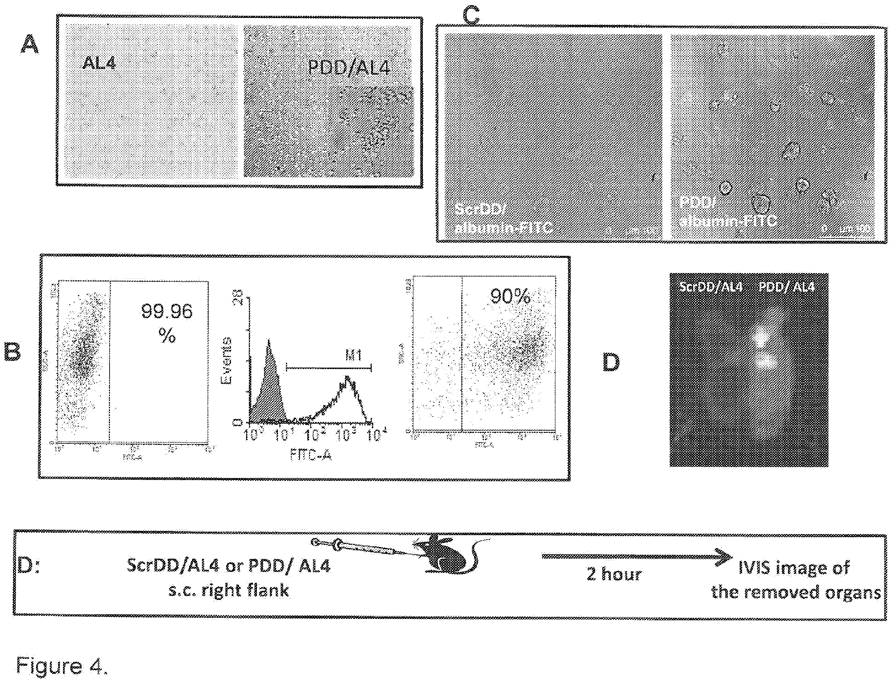

FIG. 4 shows *in vitro* and *in vivo* targeted drug delivery to macrophages, monocytes and to spleen. A. Peritoneal macrophages were collected 12 hours post ip. injections and examined under a fluorescent microscope. Five days prior to the experiment mice had received ip. injections of sterile thioglycollate as is described in the materials and methods of Example 1. B. After 90 minutes of co-culturing of human purified monocytes with either PDD/siRNA-FITC or siRNA-FITC, cells were examined by FACS. Panel C shows the proof of targeting spleen *in vivo.* IVIS images of removed spleens of mice one hour post s.c. injections of AL4 alone or PDD/AL4 complex, the image was taken 1 hour post injections as is depicted. The injections were performed in the opposite flank of the anatomical site of spleen.

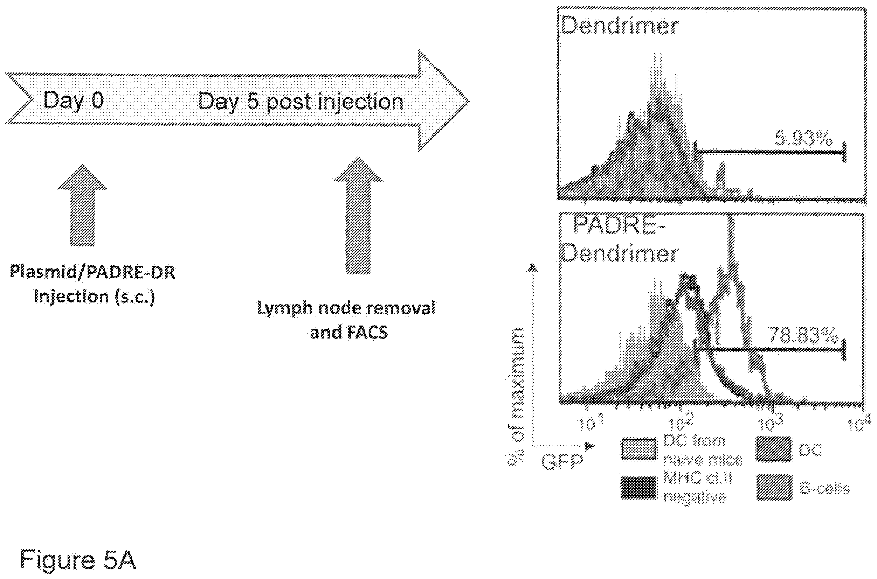

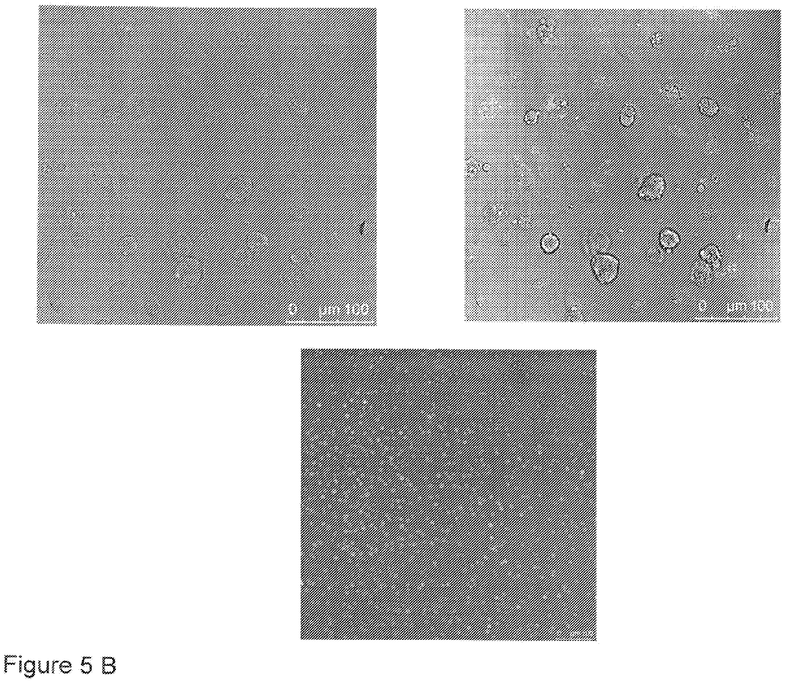

FIG. 5 shows evidence that the nanocarrier platform that targets cells expressing MHC class II as described herein specifically targets and shuttles the cargo into DCs in draining lymph nodes in the host and *in vitro.* 5A. DNA plasmid harboring GFP was mixed with the PDD in a weight ratio of 1:7. After 20 minutes incubation at room temperature, GFP plasmid/PDD complex was injected in the left flank of C57BL mice subcutaneously. Five days post injection, the draining and control lymph nodes were collected/meshed and assayed/analyzed by flow cytometry for the GFP expression in DCs as it is described in the material and methods of Example 1. 5B. Confocal microscopy indicating the presence of payload inside the cells. Albumin-FITC was mixed with PDD or with a control dendrimer coupled to random peptides at a weight ratio of 1:10. After 20 minutes incubation at room temperature confocal images at 50 nm intervals were obtained. The 0.5 micron slices through the macrophages determined that PDD/Alb-FITC at 10:1, were shuttled into the cell. Sections z31 and z39 are shown.

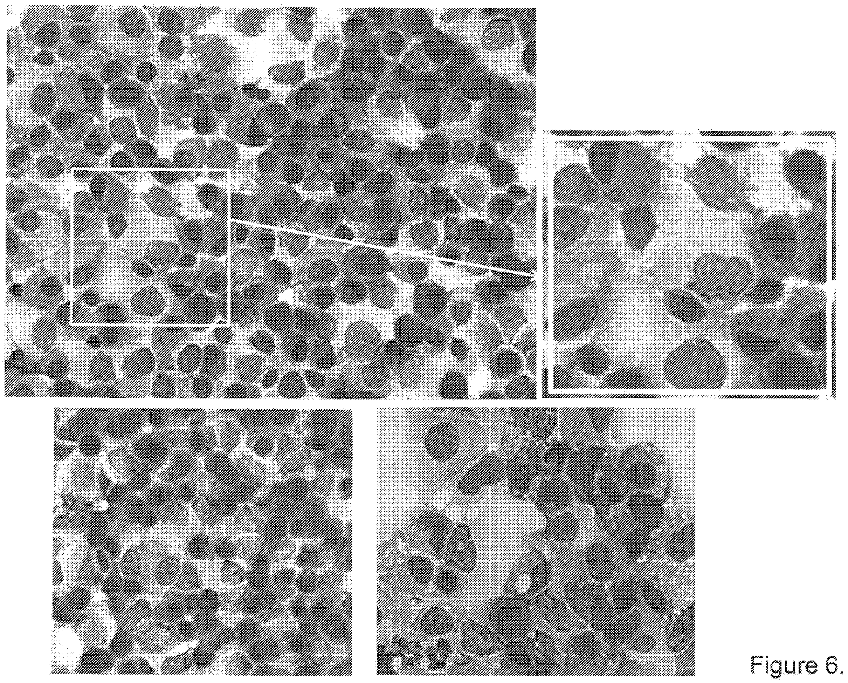

FIG. 6 is a Giemsa stain of enriched peritoneal macrophages in mice. Macrophage enriched populations were prepared by ip. injection of thioglycollateas described in materials and methods of Example 1. Purified murine macrophage enriched cells isolated from peritoneal cavity and their treatment with PDD/AmBisome®. Giemsa staining of mice enriched peritoneal before (lower left panel) or after AmB treatment (lower right panel). Macrophage-enriched populations were prepared as is described in the materials and methods of Example 1. Top panel shows the peritoneal macrophages upon 30 min incubation with PDD/AmB; macrophages were stained upon a cytospin.

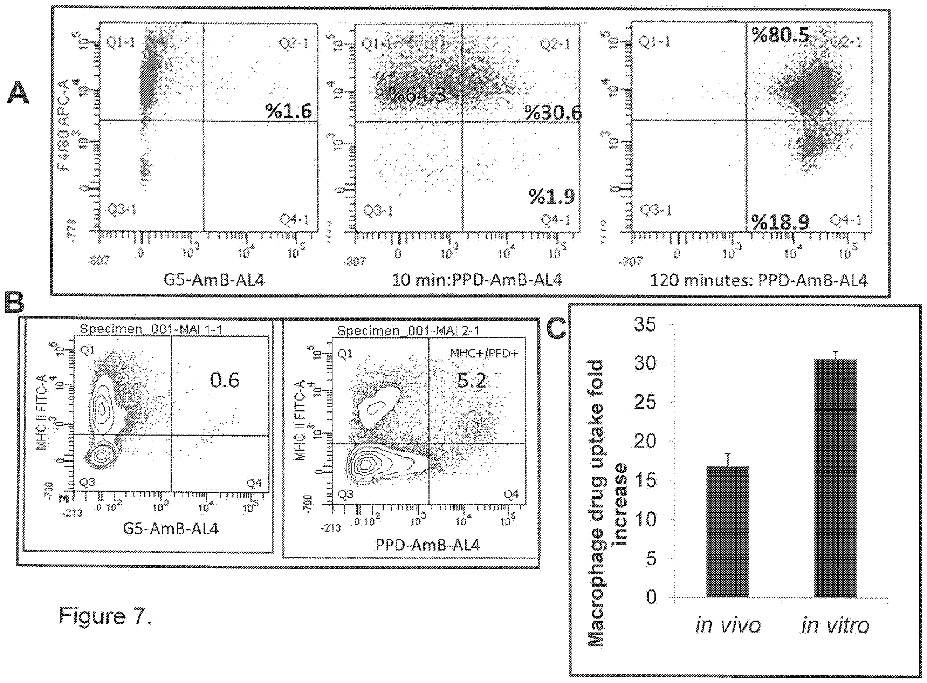

FIG. 7 shows that PDD enhances AmB uptake by macrophages. A. The delivery of AmB into macrophages after 10 or 120 min *in vitro* co-incubation with or without PDD. *In vitro* AmB delivery into macrophages upon either 10 min or 120 min *in vitro* co-incubation with PDD/AL4 or a control dendrimer, G5/AL4. B. *In vivo* AmB delivery was assessed 2 hours post i.p. injection of AmB with or without PDD by flow cytometry staining of macrophages isolated from peritoneal cavity. C. Increase in AmB uptake mediated by PDD, *in vitro* and *in vivo.*

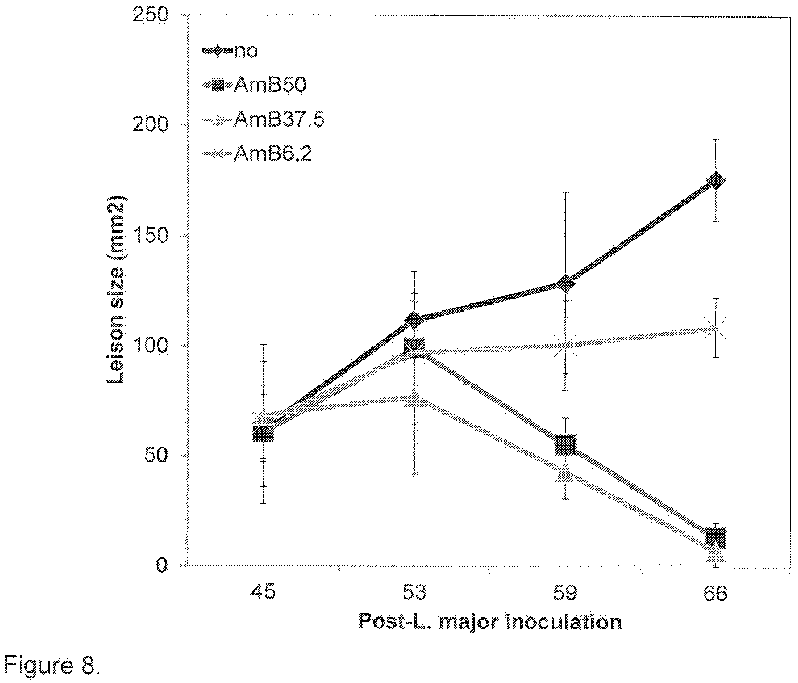

FIG. 8 is a graph showing the baseline of the efficacy of AmB doses on cutaneous Leishmaniasis in BALB/c mice. Mice (in groups of 10) received one million *L. major* parasites. Treatments compared different doses of AmB for 10 days via i.p. when the lesion sizes were between 20-70 mm² administrations.

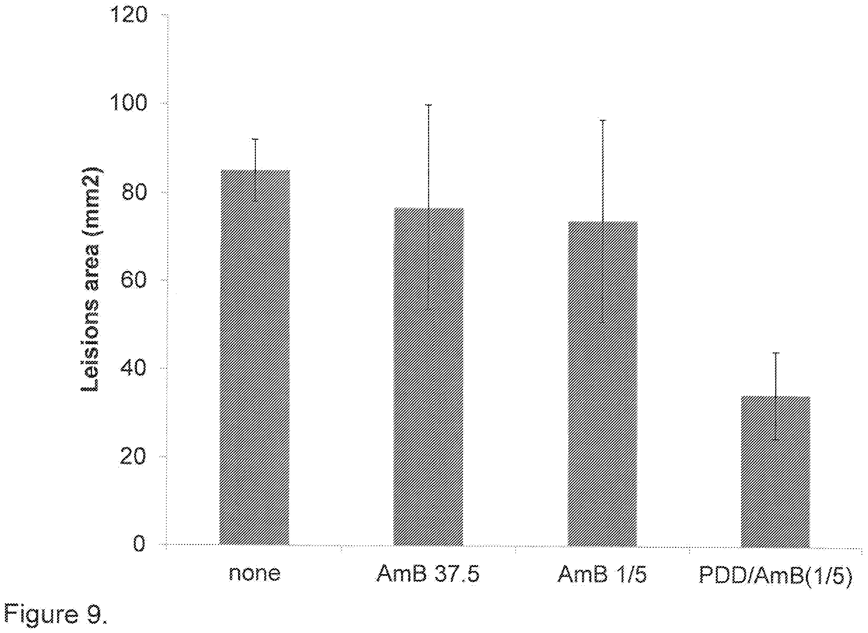

FIG. 9 is a graph showing effectiveness as early as day 7 post-therapy. The complexation of AmB with PDD results in an accelerated effect of the AmB at 1/5 the dose. Rapid acting/kinetics of AmB/PDD is shown. The treatments started on day 53 post infection. The lesion sizes are shown on day 7 post treatment.

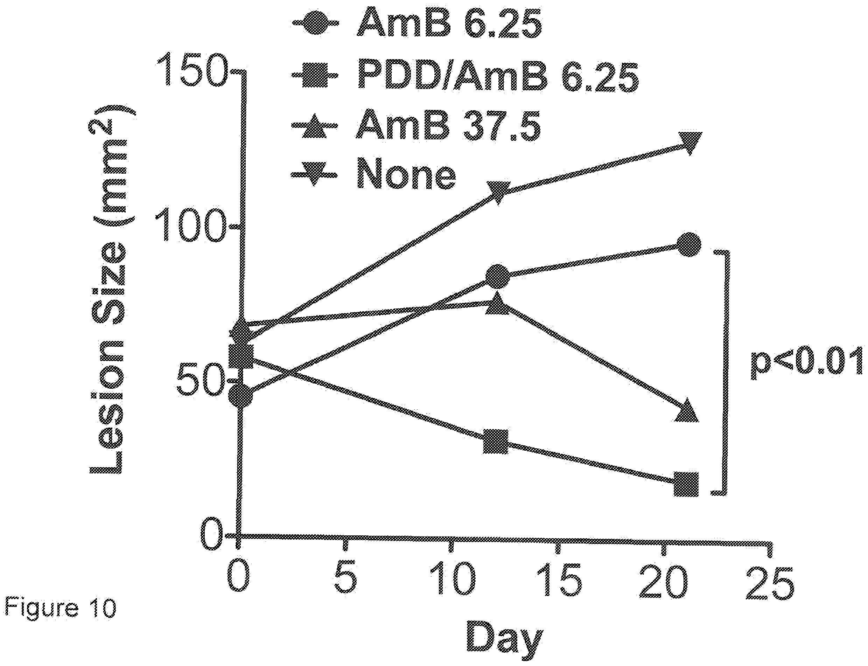

FIG. 10 is a graph showing lesion sizes in days 0, 12, and 21 post therapy. BALB/c mice bearing leishmaniasis skin lesions were either untreated (none) or treated for 10 days with AmB (6.25 mg/Kg/day), AmB (37.5 mg/Kg/day), or AmB/DRHA (AmB in complex: 6.25 mg/ Kg/ day). Mice in 5 per group received different treatments and the average ± SD of the lesion areas (mm²) are shown.

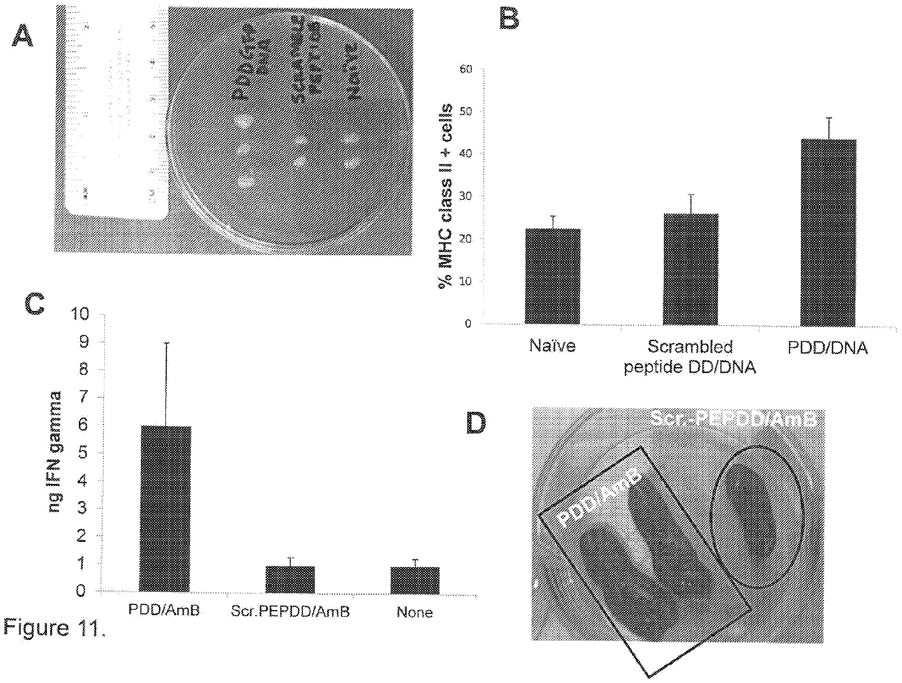

FIG. 11 shows the intrinsic immunoenhancing effects of PDD1 and PDD. A and B: Draining lymph nodes and spleens from C57BL and BALB/c mice that received i.p. injections of PDD1/AmB or Scramble (Random)-peptide-dendrimer complexed with AmB alone are shown. A transient enlargement of the draining lymph nodes and spleens of mice receiving PPD/AmB is shown. C: Mice in groups of five received either no injections or PDD1/AmB or AmB injections as described. Splenocytes of mice that were not treated, or were treated with AmB with or without PDD1 were isolated. The splenocytes were co-cultured with plasmids harboring *L. major* antigens complexed with PDD1. IFNγ was measured in the supernatant after 48 hours. Panel D shows the increased expression of MHC class II, a sign of activation of immune responses, in mice that received PDD/AmB and not in those that received the control dendrimer plus AmB.

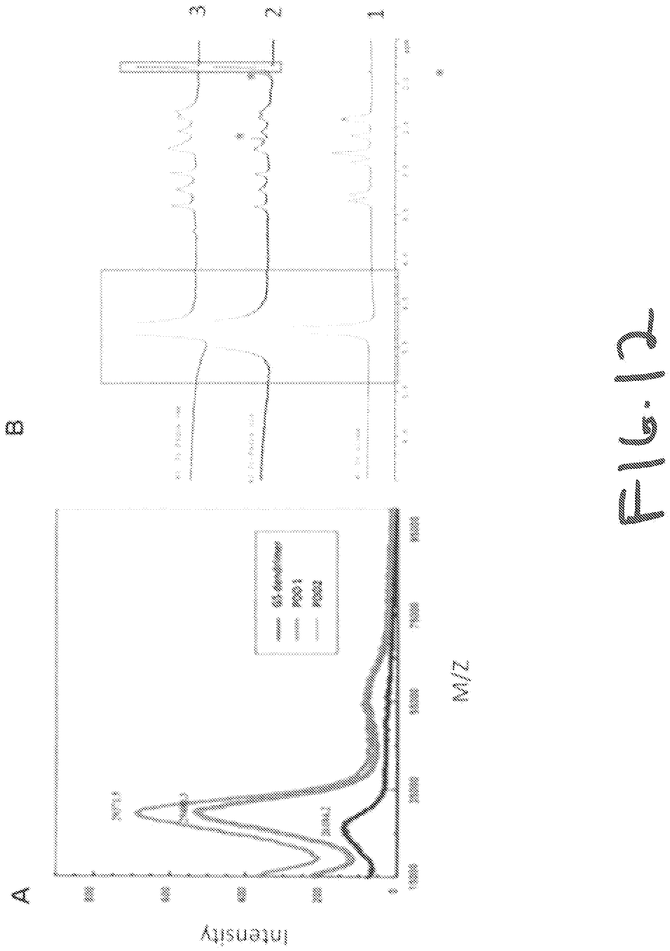

FIG. 12 shows results from an NMR analysis of the two PDD batches compared with dendrimer alone showing the consistency in the conjugation process/products.

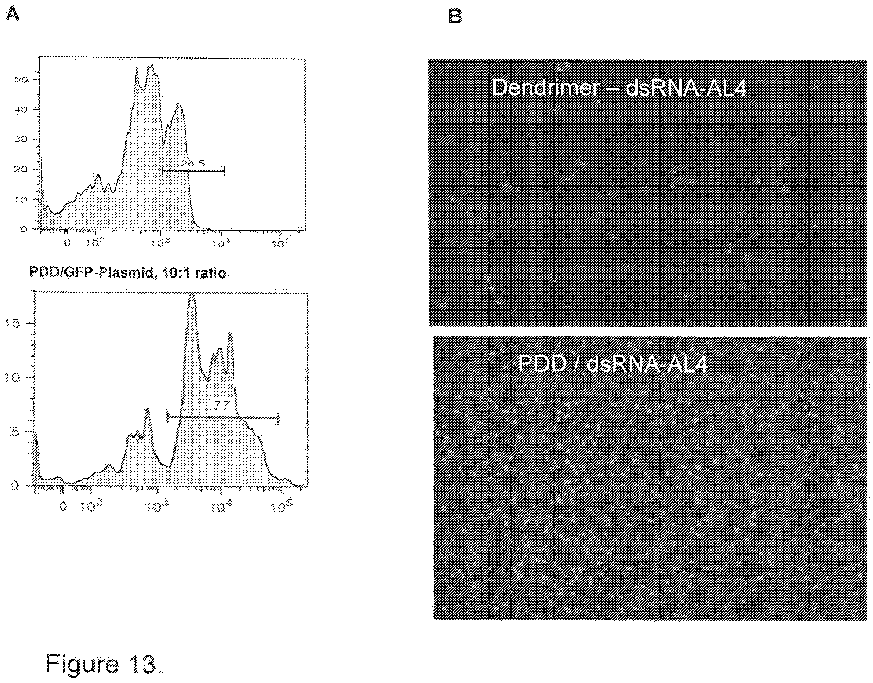

FIG. 13 shows results from an analysis comparing PDD, AmB, and PDD/AMB which suggests that the latter may be a mixture of the PDD and AmB.

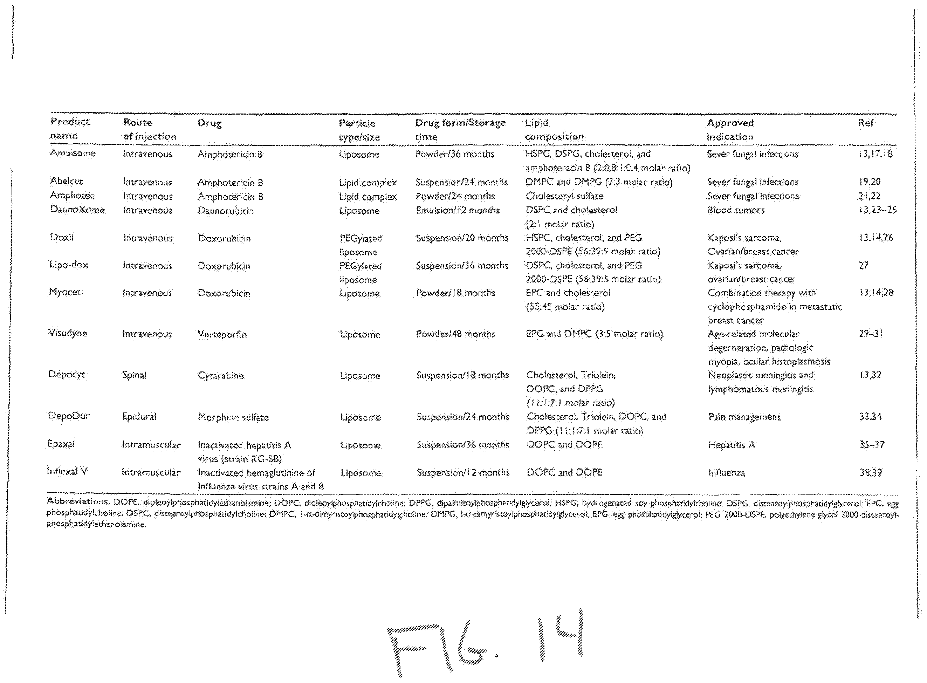

FIG. 14 is a Table listing liposome-based drugs on the market.

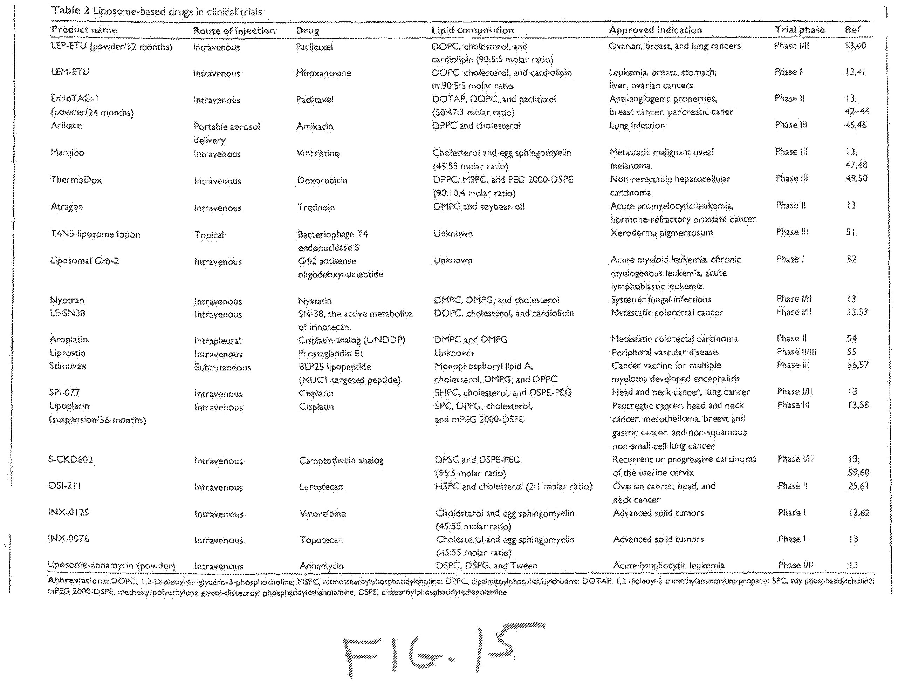

FIG. 15 is a Table listing liposome-based drugs in clinical trials.

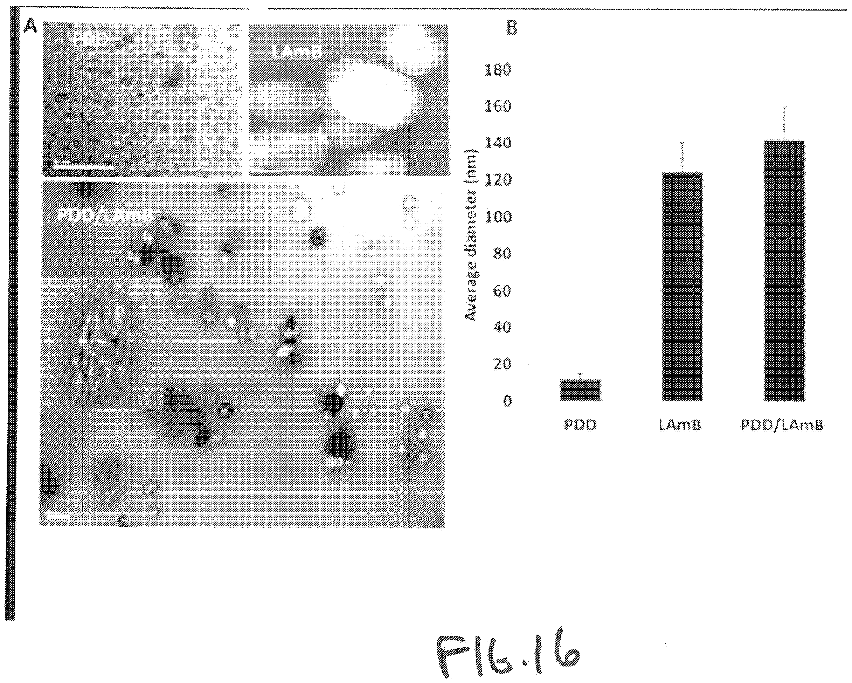

FIG. 16 is a series of images and a graph showing PDD/LAmB complexation characterizations. Figure 16A shows the TEM image of a PDD/LAmB complex. Multiple PDD particles complex with a LAmB. Figure 16B shows the average diameters of PDD, LAmB, or PDD/LAmB complexes using DLS.

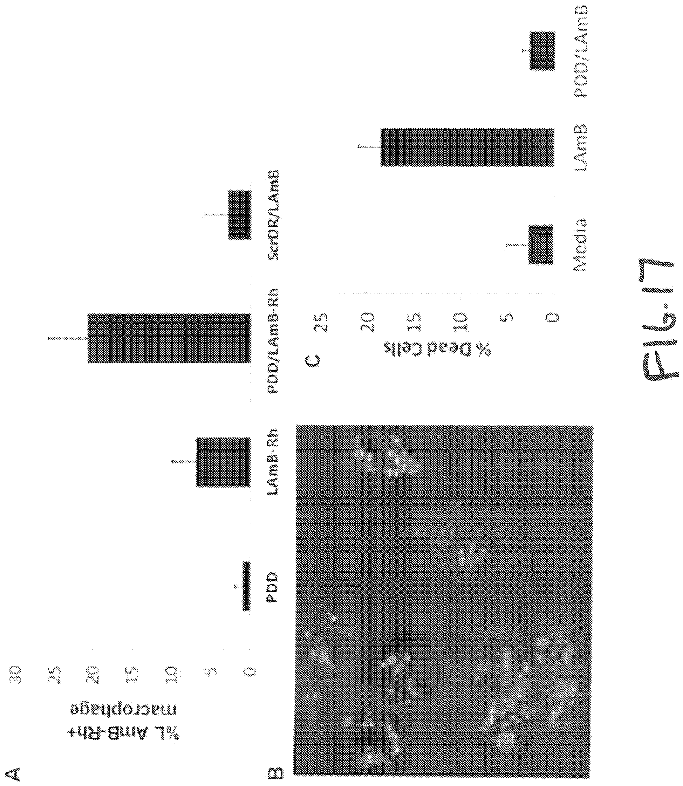

FIG. 17 is an image and a pair of graphs showing PDD/LAmB in vivo APC targeting and effect on LAmB toxicit. 17A summarizes results from a flow cytometry analysis of isolated resident peritoneal macrophages upon interaperitoneal injections of LAmB-Rh, PDD/LAmB-Rh, or controls. In vivo macrophage targeting assessed by flow cytometry analysis showed that percentage of macrophages positive for Rh delivered by PDD/LAmB-Rh is significantly (p<0.001) higher (%30+/- 5%) than that delivered by LAmB-Rh or by ScrDR/LAmB (%7+/- 3.7%). 17B shows the confocal image of the peritoneal macrophages (Z-6 of a series of 20 fluorescence images where Z was 1 µm) demonstrating that the LAmB-Rh is up-taken by macrophage. 17C shows the In vitro toxicity of L-AmB and PDD/L-AmB on HepG2 cells. L-AmB with and without PDD was added at 1 microgram per ml of HepG2 cells in the culture and MTT assay was performed as it is discussed in the materials and methods. The average percentages of death cells of 3 separate experiments are shown. L-AmB cell toxicity on HepG2 cells was reversed by PDD (p<0.007).

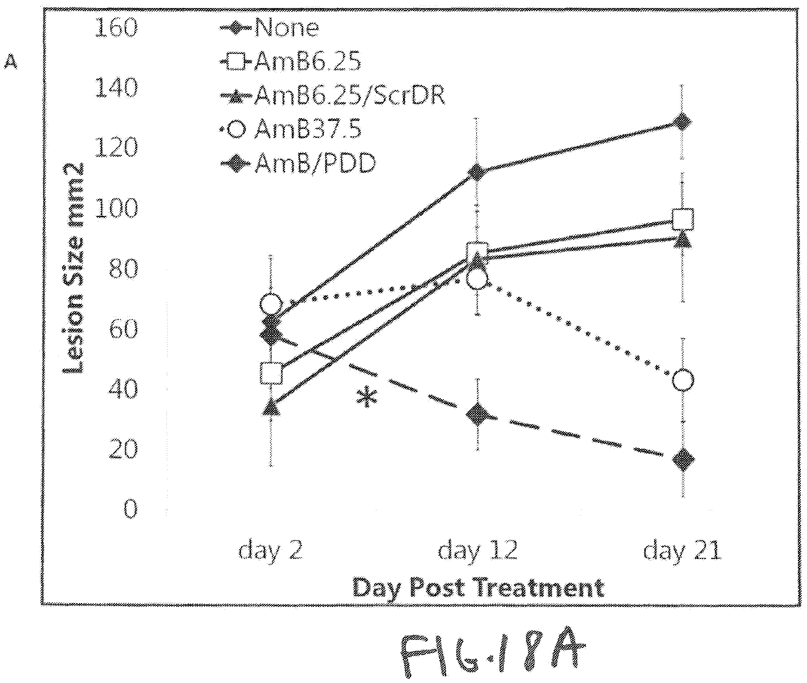

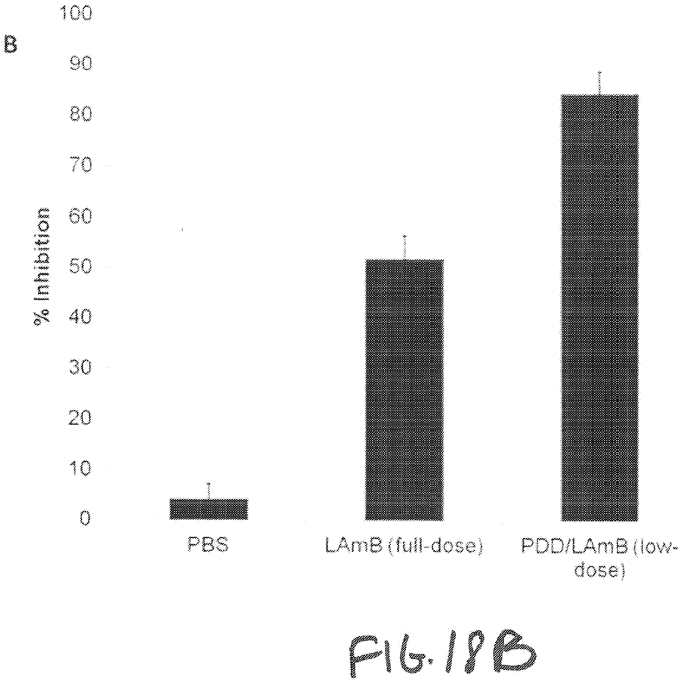

FIG. 18 is a pair of graphs showing that PDD lowers LAmB effective dose and accelerates its efficacy. 18A shows the effects of different doses and formulation of LAmB on cutaneous leishmaniasis as assessed by lesion area. Mice bearing cutaneous *L. major* lesions of similar size received either the full dose LAmB, or LAmB at a low-dose (6.25 mg/K/Day) either alone, encapsulated in a control nanoparticle (ScrDR), or in PDD1. The therapeutic effects of PDD1/LAmB low-dose (6.25 mg/k/day for 10 days) is accelerated (*) when compared to that of full-dose (37.5 mg/k/day for 10 days, *dotted line*). Fig. 18B, similar data was achieved using PDD2 engrafted LAmB showing a significantly faster effect on reducing lesion areas. 18B shows the quantitative parasite burden in spleen 12 days after treatments. QPCR was performed on the DNA obtained from known amounts of the spleen samples of mice which have been treated with either LAmB (full-dose), PDD/LAmB (low-dose) or the PBS treated group. The data is shown as the percentage of the inhibition of parasite burden in spleens of mice.

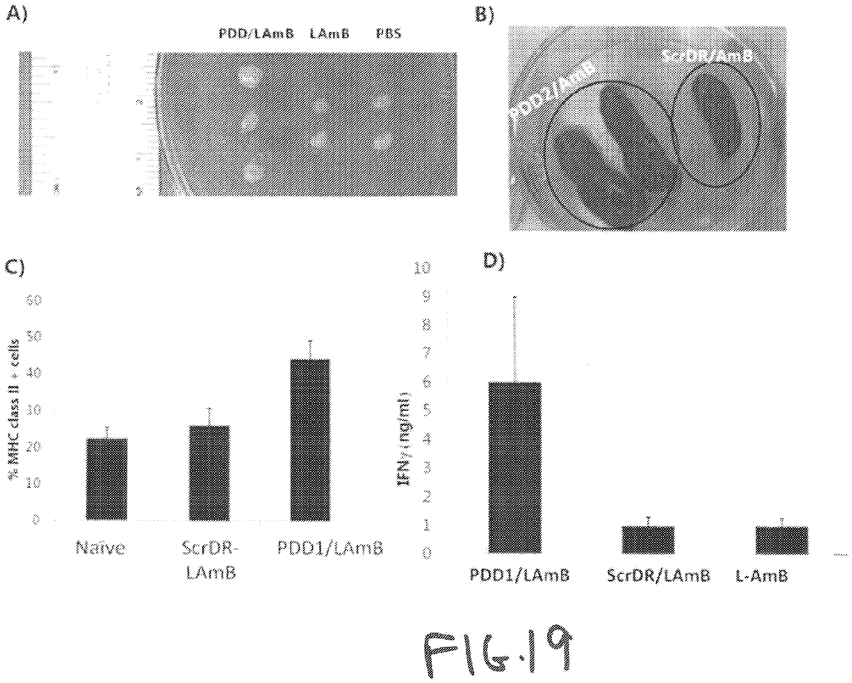

FIG. 19 is a pair of photographs and a pair of graphs showing the PDD immunopotentiating effects in infected host. 19A and 19B show the transient lymphoadenopathy (enlargement of draining lymph nodes and spleen) of infected mice receiving PDD/LAmB versus controls. 19C shows the increased MHC class II expression in the cells of draining lymph node of infected mice treated with PDD/LAmB as compared with control treatments. 19D. Mice with *L. major* lesions received i.p. treatments of PDD/LAmB (low-dose), ScrDR/LAmB (low-dose), or L-AmB (full-dose) for 10 days. Splenocytes of individual mouse were isolated and placed at 1 × 10⁶ cells/well in 96-well tissue culture plates. Splenocytes from treated animals were stimulated with either a) HA-dendrimer (40 µg) complexed with a set of plasmids (4 µg) that encode for *L. major* antigens, or b) HADR (40 µg) complexed with empty pVAX (4 µg), which served as a background and was deducted from the "a" data. IFNg measurements in the splenocyte culture supernatants were performed after a 48 hours incubation at 37°C in air with 5% CO₂, as is described in methods. Therapy with low dose LAmB significantly enhances antigen specific T cell induced IFNg when compared with full dose LAmB, in infected host (p< 0.001).

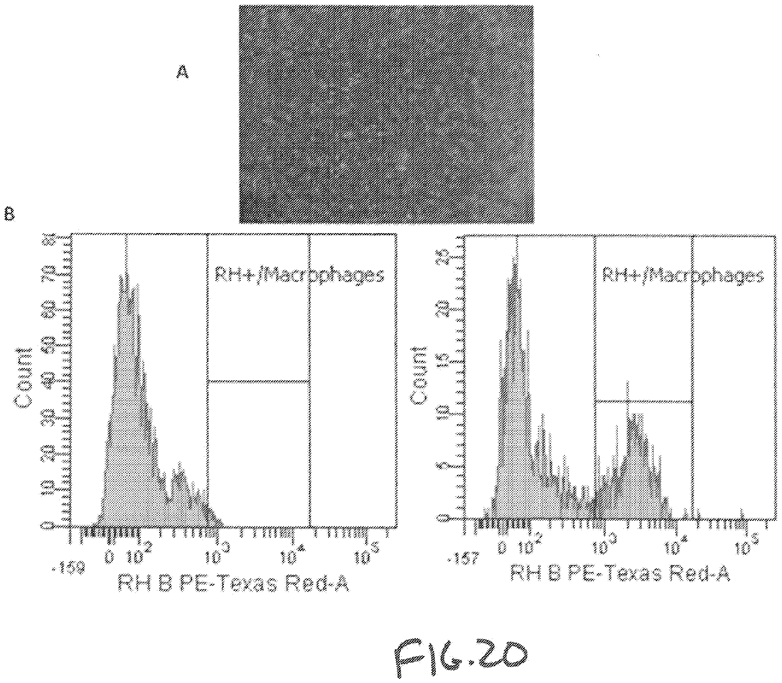

FIG. 20 is a micrograph and a pair of graphs showing LAmB Fluorescent labeling and in vivo targeting. Fig. 20 A shows the Rhodamin B encapsulated LAmB (LAmB-Rh). As is described in the material and method section, to fluorescently label L-AmB, Rhodamin B was encapsulated into L-AmB.

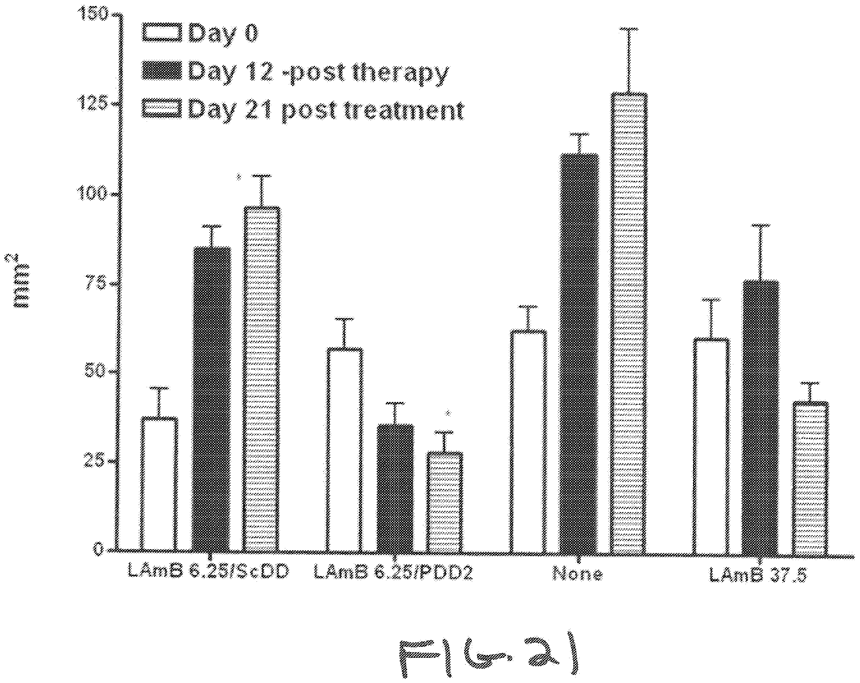

FIG. 21 is a graph showing that PDD2 results lowers effective dose and accelerates the kinetics of the therapeutic effects of the treatments. This is a similar experiment setting as was described for FIG. 16 with the exception that a second PDD (PDD2) was used. As was seen for the PDD1/LAmB, PDD2 engrafted LAmB lowered LAmB effective dose and resulted in a significantly faster effect on reducing lesion areas. PDD2 is composed of an HA helper peptide (as the APC ligand) coupled to a PAMAM dendrimer.

### DETAILED DESCRIPTION

Described herein are nanoparticle- or nanocarrier-based compositions, kits, platforms and methods for effective delivery of a therapeutic agent (e.g., a drug) *in vivo* to PAPCs or MHC class II positive cells, including cells infected with an intracellular pathogen (e.g., *Leishmania*, malaria, mycobacterium tuberculosis, HIV, etc.). A targeted delivery platform that offers targeted drug delivery to phagocytes, lowering toxicity, dose, enhancing efficacy of drugs as well as rapidness of action is described herein. In a typical composition, a charged (e.g., positively-charged), highly branched polymeric dendrimer is conjugated to 1) an MHC targeting moiety (e.g., a targeting and immunogenic peptide such as a T helper peptide (e.g., an epitope such as the PADRE peptide or Influenza HA, etc.)) for inducing an immune response to a particular pathogen in a subject and 2) to at least one anti-pathogen agent for delivery of the anti-pathogen agent (e.g., drug) into PAPCs in the subject. In another composition, the anti-pathogen agent is conjugated to the MHC targeting and immunogenic peptide rather than to the charged, highly branched polymeric dendrimer. In general, the drug, other therapeutic agent and/or cargo is encapsulated in liposomes with a net-charge opposite to that of dendrimer. For example, the charge of the dendrimer may be negative and the charge of the liposomal drug, other therapeutic agent and/or cargo may be positive. In another example, the charge of the dendrimer may be positive and the charge of the liposomal drug, other therapeutic agent and/or cargo may be negative. Drugs, other therapeutic agents and/or cargoes may be those having opposite charges to a PDD and thus complex with the PDD, or may be those encapsulated in liposomes which have an opposite charge to a PDD and thus complex with the PDD. In some embodiments, the anti-pathogen agent may be encapsulated by the charged highly branched polymeric dendrimer. The dendrimer makes a complex (conjugation) with a therapeutic agent based on the opposite charge of the dendrimer (e.g., positive) and that of the therapeutic agent (e.g., negative) or the conjugation may be a covalent chemical linkage. For example, small molecules and drugs (which may be small molecules) can be covalently bound to the dendrimer. Targeted delivery of a drug to PAPCs as described herein offers a solution to the challenges associated with current strategies for treating infection by an intracellular pathogen such as *Leishmania* by resulting in much more robust immune responses, and preventing toxicity by lowering the dose of the anti-pathogen agent (e.g., Amb). A typical nano-carrier system as described herein has positively charged amine groups that make stable complexes with the negatively charged AmB and escorts the AmB directly into PAPCs (e.g., phagocytic cells) *in vivo.* For example, in the experiments described herein, amphotericin B, which has a neutral charge and does not make a complex with a positively charged PDD, was encapsulated in a negatively charged liposome, which then complexed with positively charged PDD. Drugs, other therapeutic agents and/or cargoes therefore can be negatively charged or can be made at least partially negatively charged, or may be engrafted in negatively charged liposomes (or other carriers) to complex with PDD. However, also encompassed by the invention are negatively charged dendrimers complexed with positively charged drugs or other therapeutic agents (or positively charged carriers).

The below described preferred embodiments illustrate adaptations of these compositions, kits, platforms and methods. Nonetheless, from the description of these embodiments, other aspects of the invention can be made and/or practiced based on the description provided below.

### Biological Methods

Methods involving conventional molecular biology techniques are described herein. Such techniques are generally known in the art and are described in detail in methodology treatises such as Molecular Cloning: A Laboratory Manual, 3rd ed., vol. 1-3, ed. Sambrook et al., Cold Spring Harbor Laboratory Press, Cold Spring Harbor, N.Y., 2001; and Current Protocols in Molecular Biology, ed. Ausubel et al., Greene Publishing and Wiley-Interscience, New York, 1992 (with periodic updates). Immunology techniques are generally known in the art and are described in detail in methodology treatises such as Advances in Immunology, volume 93, ed. Frederick W. Alt, Academic Press, Burlington, MA, 2007; Making and Using Antibodies: A Practical Handbook, eds. Gary C. Howard and Matthew R. Kaser, CRC Press, Boca Raton, Fl, 2006; Medical Immunology, 6th ed., edited by Gabriel Virella, Informa Healthcare Press, London, England, 2007; and Harlow and Lane ANTIBODIES: A Laboratory Manual, Cold Spring Harbor Laboratory Press, Cold Spring Harbor, NY, 1988. Construction and use of PAMAM dendrimers is also described, for example, in Arashkia et al., Virus Genes 40 (1): 44-52, 2010; Velders et al., J Immunol. 166:5366-5373, 2001; and S. Chauhan, N. K. Jain, P. V. Diwan. (2009) Pre-clinical and behavioural toxicity profile of PAMAM dendrimers in mice. Proceedings of the Royal Society A: Mathematical, Physical and Engineering Sciences (Online publication date: December 3, 2009).

### Platform for Targeting Drugs to PAPCs

Dendrimers are an ideal drug/multi-nucleic acids delivery candidate as they provide structural control over size and shape (cargo-space), are biocompatible (non-toxic and nonimmunogenic), have precise scaffolding properties, have a well-defined surface-modifiable functionality for specific targeting moieties, have the ability for cellular adhesion and endocytosis and delivery into the cytoplasm or nucleus, have acceptable biodegradation (the ability to safely degrade within the body), and are associated with easy and consistently reproducible (clinical grade) synthesis. Described herein are novel dendrimer-based compositions and platforms that when complexed with a therapeutic agent, such as a drug of interest, target the therapeutic agent (e.g., drug) primarily to phagocytic cells via MHC class II molecules expressed on the surface of such cells. These compositions and platforms include, for example, the combined use of a cationic PAMAM dendrimer (e.g., peptide-derivatized dendrimer PAMAM G5 or any polymer), and one or more MHC class II targeting peptides (e.g., PADRE or any universal T helper epitope). This is important for drug delivery into macrophages, monocytes, and dendritic cells and when the drugs are against obligatory intracellular parasites/pathogens such as *Leishmania,* malaria, HIV, or mycobacterium tuberculosis. A drug (therapeutic agent) may be complexed to the dendrimer-PADRE by incubation in room temperature or be coupled via direct conjugation of drug(s) to the platform, e.g., dendrimer-PADRE. Alternatively, the drug may also be coupled to the PADRE or other universal T helper epitopes. In some embodiments, the drug is encapsulated by the dendrimer-PADRE.

Examples of therapeutic agents include but are not limited to drugs, toxins, iRNA, siRNA, microRNA, antibodies, polypeptides, peptides, nucleic acids, cytokines, enzymes, hormones, clotting factors, immunotherapeutics including vaccines, (humanized monoclonal antibodies) or any combination of such agents. Examples of drugs include AmBisome®, Amphotericin B, Taxol® (paclitaxel) (Bristol-Myers Squibb), melphalan, prednisone, thalidomide (MPT), Velcade® (bortezomib) (Millenium Pharmaceuticals), lenalidomide, dexamethasone, drugs that target intracellular pathogens including Clofazimine, Amoxacillin plus Clavulonic acid, Streptomycin, or Capreomycin, nevirapine, zidovudine, lamivudine ritonavir and lopinavir. As is the case with AmB, a drug or other therapeutic agent may be complexed with a liposome or other type of vesicle. The compositions described herein may be used with adjuvants such as (but not limited to) Poly I:C which is negatively charged and makes a complex with a nanoparticle platform as described herein.

In a typical embodiment of delivering an anti-pathogen agent to PAPCs, an approved marketed drug wrapped in liposomes is used. For example, AmBisome® is a liposomal Amphotericin B where the Amphotericin B is wrapped in a 60-70 nm diameter DSPG monolayer liposome, and shows a higher LD₅₀ and better safety profile than conventional amphotericin B. AmB contains Hydrogenated soy phosphatidylcholine, vitamin E, cholesterol, and Distearoylphosphatidylglycerol. The net negative charge of the external surface of AmB increases affinity for fungal and mammalian cells. The liposome is useful for preventing the release of drug and limiting the off-target interaction of amphotericin B. Cholesterol in the platform increases the stability of the drug. Methods of preparing and using liposomes and other nanocarriers are well known in the art, and are described in, for example, Multifunctional Nanoparticles for Drug Delivery Applications: Imaging, Targeting, and Delivery (Nanostructure Science and Technology), Sonke Svenson & Robert K. Prud'homme, editors, Springer, 2012 edition; Beija et al., Trends Biotechnol. Jun 4, 2012; Parveen et al., Nanomedicine. Feb;8(2):147-66, 2012; and Puri et al., Crit Rev Ther Drug Carrier Syst. 26(6):523-80, 2009.

Dendritic cells and macrophages are major PAPCs that are central in mounting protective immune responses against cancer and the many parasites, bacteria, and viruses. They also dictate the tolerogenic immutherapies for autoimmune disorders, including diabetes. B cells also have ability to present antigens and a typical pathogen that infects human B cells is EBV. Examples of pathogens that can be treated using the compositions, methods, kits and platforms described herein include but are not limited to, pathogenic parasitic, bacterial, fungal, or viral organisms. Examples include:

| |
|---|
| *Leishmania* species (e.g., *L. major, L. tropica, L. aethiopica, L. mexicana, L. donovani, L. infantum* syn. *L. chagas*), |
| *Streptococcus* species, |
| *Candida* species, |
| *Brucella* species, |
| *Salmonella* species, |
| *Shigella* species, |
| *Pseudomonas* species, |
| *Bordetella* species, |
| *Clostridium* species, |
| Norwalk virus, |
| *Bacillus anthracis,* |
| *Mycobacterium tuberculosis,* |
| Human immunodeficiency virus (HIV) |
| *Chlamydia* species, |
| human Papillomaviruses, |
| Influenza virus, |
| Paramyxovirus species, |
| Herpes virus, |
| Cytomegalovirus, |
| Varicella-Zoster virus, |
| Epstein-Barr virus, |
| Hepatitis viruses, |
| *Plasmodium* species (e.g., *Plasmodium(p) falciparum*, *P. malariae*, *P. ovale, P. vivax* and *P. knowlesi)*, |

In one embodiment, the moiety capable of binding to MHC class II is a Pan-DR epitope, e.g., PADRE. PADRE is an artificially designed peptide that binds to the majority of murine and human MHC Class II molecules, and conjugating PADRE peptides to dendrimers (e.g., a PADRE-derivatized dendrimer) makes the resultant complex or conjugate a ligand for PAPCs that express high levels of MHC class II. PADRE is a synthetic, non-natural T helper epitope [AKchxAVAAWTLKAAA (chxA = cyclohexylalanine) (SEQ ID NO: 1)]. When fused to the surface of the dendrimer, PADRE will bind and activate primarily cells that have MHC class II including all PAPCs. Several PADRE epitopes (e.g., 2, 3, 4, 5, etc.) can be attached to each dendrimer. The attachment is done with suitable spacers to preserve the binding properties of the peptide that give rise to its immunogenic properties. A linker or spacer molecule may be used in conjugating a therapeutic agent to the dendrimer conjugates described herein. Spacers may be any combination of amino acids including AAA, KK, GS, GSGGGGS (SEQ ID NO: 2), RS, or AAY. As used herein, the terms "linker" or "spacer" mean the chemical groups that are interposed between the dendrimer and the surface exposed molecule(s) such as the MHC class II ligand, CD4+ T helper epitope, and therapeutic agent (e.g., anti-pathogen drug) that is conjugated or bound to the dendrimer (e.g., PADRE-dendrimer) and the surface exposed molecule(s). Preferably, linkers are conjugated to the surface molecule at one end and at their other end to the nanoparticle (e.g., PADRE-dendrimer). Linking may be performed with either homo- or heterobifunctional agents, i.e., SPDP, DSS, SIAB. Methods for linking are disclosed in PCT/DK00/00531 (WO 01/22995) to deJongh, et al.) Cleavable linkers may also be used in the compositions and methods described herein. For example, drug molecules may be coupled to targeted dendrimer (PDD) using a pH sensitive cleavable spacer (e.g., GFLG (SEQ ID NO: 3)) that releases the drug inside the cells. GFLG (SEQ ID NO: 3) is a tetrapeptide spacer, composed of glycylphenylalanylleucylglycine (SEQ ID NO: 3).

In another embodiment, moiety capable of binding to MHC Class II is influenza HA. In one embodiment, the moiety is a T helper epitope or any other epitope that activates or contributes to activation of CD4+ T helper cells. T helper epitope activation of CD4 + T helper cells is required for the expansion and stimulation of CD8 T cells as well as for antibody production by B cells, both of which are essential for induction of protective immune responses against infectious agents.

The compositions, methods, platforms and kits described herein have both prophylactic and treatment applications, i.e., can be used as a prophylactic to prevent onset of a disease or condition in a subject, as well as to treat a subject having a disease or condition. A composition or targeted delivery platform as described herein can be used to reduce the growth of or eliminate any infectious pathogen, as well as mount an immune response against any infectious pathogen.

The dendrimer-based platform described herein provides several advantages. The platform targets PAPCs via its MHC class II ligand, binds and penetrates the cell membrane by its highly positively-charged outer membrane, is safe and easy to scale up for high-volume production, and acts as a strong adjuvant due to the nature of modifications on the molecule. In a typical embodiment, the at least one dendrimer is a G5 PAMAM dendrimer that is a highly branched polymeric macromolecule and an ideal excipient for its enhanced solubility. Inclusion of a universal T helper agonist, e.g., PADRE, which binds to the flank of the MHC class II molecules, results in an opsinizing dendrimer complex for PAPCs as well as helper T cells. This alteration changes an inert and weak dendimer to a robust immune modulator.

In some embodiments, a polymeric particle, e.g., a biodegradable polymeric particle is used in place of a dendrimer. The polymeric particle can be charged (e.g., positively-charged, negatively-charged) or have no charge and the MHC targeting peptide (e.g., universal T helper peptide, CLIP, etc.) and the at least one therapeutic agent can be conjugated to the polymeric particle.

### Synthesis of Dendrimers Conjugated to Therapeutic Agents (Drugs)

Described herein are dendrimers having conjugated thereto at least one universal T helper peptide (e.g., an epitope such as the PADRE peptide or Influenza HA) and a therapeutic agent (e.g., an anti-pathogen drug), wherein the at least one universal T helper peptide is conjugated to the exterior surface of the dendrimer such that the at least one T helper peptide specifically binds to PAPCs, and the therapeutic agent is encapsulated by the dendrimer or conjugated to the exterior surface of the dendrimer. Dendrimers can be prepared and conjugated to a T helper peptide (e.g., an epitope such as the PADRE peptide or Influenza HA) and bound to or complexed with a therapeutic agent using any suitable method. For example, see FIG. 1 for a schematic demonstrating the synthesis of PADRE-dendrimer. Methods of producing and using dendrimers are well known in the art and are described, for example, in Zhang J-T et. al. Macromol. Biosci. 2004, 4, 575-578, and U.S. Patent Nos. 4,216,171 and 5,795,582. See also: D.A. Tomalia, A.M. Naylor, and W.A. Goddard III, "Starburst Dendrimers: Molecular-Level Control of Size, Shape, Surface Chemistry, Topology, and Flexibility from Atoms to Macroscopic Matter", Angew. Chem. Int. Ed. Engl. 29 (1990), 138-175. In the experiments described herein, PAMAM dendrimers were used. However, any suitable positively charged, highly branched polymeric dendrimer can be used. Examples of additional positively charged, highly branched polymeric dendrimers include poly(propylene imine) (PPI) dendrimers or, more generally, any other dendrimers with primary amine groups on their surfaces. Depending on the type of therapeutic agent, more than one (e.g., a plurality) positively charged, highly branched polymeric dendrimers may be conjugated to a single therapeutic agent. For example, multiple PDD may bind to one AmBisome®, as AmBisome® is 100 nm and a PDD as described herein is approximately 8 nm.

In one embodiment, dendrimers are conjugated to at least one PADRE peptide (e.g., 2, 3, 4, 5, etc.) and a drug for treating an infection by an intracellular pathogen. The PADRE-dendrimers (PADRE-derivatized dendrimers) described herein can be prepared by any suitable method. Methods of making and using PADRE are known in the art. See, for example, U.S. Patent No. 5,736,142. PADRE peptides can be prepared according to the methods described in U.S. Patent No. 5,736,142, for example, or they can be purchased (e.g., from Anaspec, Inc., Fremont, CA). Because of their relatively short size, the PADRE peptides can be synthesized in solution or on a solid support in accordance with conventional techniques. Various automatic synthesizers are commercially available and can be used in accordance with known protocols. Alternatively, recombinant DNA technology may be employed wherein a nucleotide sequence which encodes a T helper epitope is inserted into an expression vector, transformed or transfected into an appropriate host cell and cultivated under conditions suitable for expression. These procedures are generally known in the art, as described generally in Sambrook et al., (*supra*), which is incorporated herein by reference. PADRE peptides as described herein may include modifications to the N- and C-terminal residues. As will be well understood by the artisan, the N- and C-termini may be modified to alter physical or chemical properties of the peptide, such as, for example, to affect binding, stability, bioavailability, ease of linking, and the like. The PADRE peptides described herein may be modified in any number of ways to provide desired attributes, e.g., improved pharmacological characteristics, while retaining substantially all of the biological activity of the unmodified peptide.

In the experiments described herein, the PADRE-dendrimer conjugate was made by simple amide coupling between the -COOH terminus of the PADRE peptide and one of the dendrimer amine groups. The PADRE peptide (Ac-D-Ala-Lys-Cha-Val-Ala-Ala-Trp-Thr-Leu-Lys-Ala-Ala-Ala-D-Ala-Ahx-Cys-OH (SEQ ID NO: 4)) (Ac= acetylated; D-Ala = D-alanine; Cha = cyclohexylalanine; Ahx = aminohexanoic acid) was purchased from Twentyfirst Century Biochemicals, Inc. (Marlboro, MA) in its acetylated form in order to protect the amine terminus and prevent its reaction. The purchased peptide had a minimum purity of 95%. The amide coupling reaction was carried out under standard conditions in DMF solution or in MBS. There are variants of PADRE, and all such variants are encompassed by the compositions and methods described herein.

In order to control the number of PADRE epitopes attached to the surface of each dendrimer, a 2:1 peptide/dendrimer challenge ratio was used in the reaction, seeking attachment of just a few peptides per dendrimer in order to keep most of the amine groups free to develop large positive charges on the dendrimer. In a typical embodiment, a plurality of PADRE-dendrimer conjugates as described herein will be a distribution of dendrimers containing 0, 1, 2, 3, etc., PADREs (or other peptide) attached thereto. Relative populations are expected to follow the Poisson distribution. The PADRE peptide variants including aKXVAAWTLKAAa (SEQ ID NO: 5) bind with high or intermediate affinity (IC₅₀<1,000 nM) to 15 out of 16 of the most prevalent HLA-DR molecules ((Kawashima et al., Human Immunology 59:1-14 (1998); Alexander et al., Immunity 1:751-761 (1994)). However, other peptides which also can bind MHC class II and activate CD4 T helper cells in most humans may also be used to tag the dendrimer.

Examples of APC targeting peptides include but are not limited to: tetanus toxoid (TT) peptide 830-843; the "universal" epitope described in Panina-Bordignon et al., (Eur. J. Immunology 19:2237-2242 (1989)); and the following peptides that react with MHC class II of most human HLA, and many of mice: aKFVAAWTLKAAa (SEQ ID NO: 6), aKYVAAWTLKAAa (SEQ ID NO: 7), aKFVAAYTLKAAa (SEQ ID NO: 8), aKXVAAYTLKAAa (SEQ ID NO: 9), aKYVAAYTLKAAa (SEQ ID NO: 10), aKFVAAHTLKAAa (SEQ ID NO: 11), aKXVAAHTLKAAa (SEQ ID NO: 12), aKYVAAHTLKAAa (SEQ ID NO: 13), aKFVAANTLKAAa (SEQ ID NO: 14), aKXVAANTLKAAa (SEQ ID NO: 15), aKYVAANTLKAAa (SEQ ID NO: 16), AKXVAAWTLKAAA (SEQ ID NO: 17), AKFVAAWTLKAAA (SEQ ID NO: 18), AKYVAAWTLKAAA (SEQ ID NO: 19), AKFVAAYTLKAAA (SEQ ID NO: 20), AKXVAAYTLKAAA (SEQ ID NO: 21), AKYVAAYTLKAAA (SEQ ID NO: 22), AKFVAAHTLKAAA (SEQ ID NO: 23), AKXVAAHTLKAAA (SEQ ID NO: 24), AKYVAAHTLKAAA (SEQ ID NO: 25), AKFVAANTLKAAA (SEQ ID NO: 26), AKXVAANTLKAAA (SEQ ID NO: 27), AKYVAANTLKAAA (SEQ ID NO: 28), FNNFTVSFWLRVPKVSASHLE (SEQ ID NO: 29), SSVFNVVNSSIGLIM (SEQ ID NO: 30), SKMRMATPLLMQ (SEQ ID NO: 31), and QYIKANSKFIGITEL (SEQ ID NO: 32), (a = D-alanine, X = cyclohexylalanine). Such peptides bind to MHC class II molecules present on T cells of more than 95% of all humans.

The product was purified by dialysis against pure water for at least 24 h and then dried under vacuum. The collected product, a clear oil, was characterized by ¹H NMR, UV-Vis and MALDI-TOF mass spectroscopy. The NMR spectra of the PADRE-dendrimer conjugate shows large peaks corresponding to the dendrimer protons and a small set of peaks for the peptide protons. The MALDI-TOF mass spectrum of the PADRE-dendrimer conjugate shows a peak at a m/z ratio ca. 3,000 units higher than the peak observed for the dendrimer on its own. The excess mass corresponds to approximately 2 peptide epitopes. The UV-Vis spectrum of the conjugate shows a clear absorption in the wavelength range where tryptophan absorbs.

Dendrimers that are conjugated to MHC targeting peptides (e.g., T helper epitopes) other than PADRE are typically prepared by a method similar to that described above for PADRE-derivatized dendrimers. For example, the acid terminus of the peptide can be covalently attached to one of the amine groups on the dendrimer surface by a number of well-known synthetic methods, such as amidation using carbodiimides as activating reagents. As another example, attachment of these peptides to amino-terminated dendrimers is performed using two synthetic routes. The amino terminus of the peptide epitope is protected by acetylation. The first route uses the carboxylic acid of the terminal cysteine residue to achieve attachment via standard amidation chemistry. The second route takes advantage of the cysteine's thiol (if present on the peptide, otherwise may be added) to react it with the alkene groups added to the dendrimer surface by previous treatment with maleimide. Both routes allow the functionalization of dendrimers with epitopes. Up to several peptide epitopes (e.g., 2, 3, 4, 5, 6, etc.) per dendrimer will enhance the targeting property of the drug delivery agents. However, it is important to leave a large number of unreacted amine groups so that the dendrimer will acquire a large positive charge via protonation at physiological pH values. Dendrimers as described herein can be conjugated to any T helper epitope (e.g., Influenza HA and Pan DRT helper epitope).

Generally, generation-5 (G5) dendrimers are used in the compositions, kits, platforms and methods described herein. However, other generation dendrimers (see Table 1) can be used.

**Table 1 PAMAM Dendrimers**

| Generation | Molecular Weight | Diameter (nm) | Surface Groups |
|---|---|---|---|
| 0 | 517 | 1.5 | 4 |
| 1 | 1,430 | 2.2 | 8 |
| 2 | 3,256 | 2.9 | 16 |
| 3 | 6,909 | 3.6 | 32 |
| 4 | 14,215 | 4.5 | 64 |
| 5 | 28,826 | 5.4 | 128 |
| 6 | 58,0548 | 6.7 | 256 |

### Methods of Delivering a Therapeutic Agent to PAPCs and Inducing an Immune Response

Described herein are methods of delivering a therapeutic agent for treating or preventing an infection by a pathogen specifically to PAPCs in a subject (e.g., human), and inducing an immune response (e.g., activation of CD4 T helper cells, production of monoclonal antibodies) against the pathogen. A typical method includes the steps of: administering to the subject a composition including at least one dendrimer (e.g., a charged highly branched polymeric dendrimer) having conjugated thereto at least one moiety capable of binding to MHC Class II molecules (e.g., universal T helper peptide) and at least one therapeutic agent, wherein the at least one moiety capable of binding to MHC Class II molecules and the at least one therapeutic agent are conjugated to the exterior surface of the dendrimer such that the at least one moiety capable of binding to MHC Class II molecules specifically binds to PAPCs. The composition is capable of inducing an immune response when administered to a subject, and is administered in an amount effective to induce MHC class II mediated activation of helper T cells, induce production of monoclonal antibodies against the pathogen, and inhibit the growth of or eliminate the pathogen in the subject. Generally, the at least one dendrimer is a G5 dendrimer (a PAMAM dendrimer). The at least one moiety capable of binding to MHC Class II molecules is typically one of: a Pan-DR T helper epitope (PADRE), and a T helper epitope of influenza HA molecule.

In a typical method, the therapeutic agent is an anti-pathogen agent (e.g., AmBisome® at a concentration of about 1 mg/kg to about 50 mg/kg for a single dose. In one example, the therapeutic agent is AmBisome® and at least a plurality of the professional APCs are infected with *Leishmania.* In such an embodiment, the MHC class II mediated activation of helper T cells is specific for *Leishmania,* and administration of the composition induces increased production of IFN-γ in splenocytes of the infected subject. If the subject is infected with *Leishmania,* the subject may have at least one lesion. In such an embodiment, administration of the composition results in a reduction in size or elimination of the at least one lesion.

Administration of the composition generally results in no local adverse reactions in the subject. The compositions, platforms, kits and methods described herein can be utilized with any suitable subject, including invertebrate and vertebrate subjects. In a typical embodiment, a subject to be treated is an animal such as a mammal (e.g., human beings, rodents, dogs, cats, goats, sheep, cows, horses, etc.). A human patient suffering from or at risk of contracting an infectious disease (e.g., an intracellular pathogen infection) is a typical subject. For example, the subject is a human, and the intracellular pathogen is one of: *Leishmania*, malaria, tuberculosis, and HIV.

### Kits for Delivering Drugs to APCs

Kits for treating or preventing infection by an intracellular pathogen (e.g., *Leishmania*) in a subject are described herein. A typical kit includes: a composition including at least one dendrimer having conjugated thereto at least one moiety capable of binding to MHC Class II molecules (e.g., universal T helper peptide) and at least one therapeutic agent, wherein the at least one moiety capable of binding to MHC Class II molecules and the at least one therapeutic agent are conjugated to the exterior surface of the dendrimer such that the at least one moiety capable of binding to MHC Class II molecules specifically binds to PAPCs and wherein the composition is capable of inducing an immune response when administered to a subject; at least one buffer, instructions for use, and packaging. Lyophilized PDD or any other feasible form of PDD (universal T helper epitope such as PADRE conjugated to dendrimer) may be included in a kit. In one example of a kit, the kit includes the therapeutic agent (e.g., a drug) and a buffer composed of physiological saline, phosphate buffer saline, or OptiMem, that has a physiological pH and is buffered.

### Administration of Compositions

The compositions described herein may be administered to invertebrates, animals, and mammals (e.g., dog, cat, pig, horse, rodent, non-human primate, human) in any suitable formulation. For example, a composition including a PADRE-dendrimer conjugated to a therapeutic agent may be formulated in pharmaceutically acceptable carriers or diluents such as physiological saline or a buffered salt solution. Suitable carriers and diluents can be selected on the basis of mode and route of administration and standard pharmaceutical practice. A description of exemplary pharmaceutically acceptable carriers and diluents, as well as pharmaceutical formulations, can be found in Remington's Pharmaceutical Sciences, a standard text in this field, and in USP/NF. Other substances may be added to the compositions to stabilize and/or preserve the compositions.

The compositions described herein may be administered to a subject (e.g., mammals) by any conventional technique. Typically, such administration will be parenteral (e.g., intravenous, subcutaneous, intramuscular, intraperitoneal, oral, nasal, or intrathecal introduction. The compositions may also be administered directly to a target site. The compositions may be administered in a single bolus, multiple injections, or by continuous infusion (e.g., intravenously, by peritoneal dialysis, pump infusion). For parenteral administration, the compositions are preferably formulated in a sterilized pyrogen-free form. In therapeutic applications, the compositions described herein are administered to an individual already infected with the pathogen of interest (e.g., *Leishmania*)*.* In prophylactic applications, the compositions described herein are administered to an individual at risk of developing (e.g., genetically predisposed to, or environmentally exposed to) or contracting an infectious disease (i.e., infected with a pathogen of interest).

### Effective Doses

The compositions described herein are preferably administered to a subject (e.g., invertebrates, animals, mammals (e.g., dog, cat, pig, horse, rodent, non-human primate, human)) in an effective amount, that is, an amount capable of producing a desirable result in a treated subject (e.g., protection against infectious disease(s)). Such a therapeutically effective amount can be determined as described below.

Toxicity and therapeutic efficacy of the compositions described herein can be determined by standard pharmaceutical procedures, using either cells in culture or experimental animals to determine the LD₅₀ (the dose lethal to 50% of the population). The dose ratio between toxic and therapeutic effects is the therapeutic index and it can be expressed as the ratio LD₅₀/ED₅₀. Those compositions that exhibit large therapeutic indices are preferred. While those that exhibit toxic side effects may be used, care should be taken to design a delivery system that minimizes the potential damage of such side effects. The dosage of preferred compositions lies preferably within a range that includes an ED₅₀ with little or no toxicity. The dosage may vary within this range depending upon the dosage form employed and the route of administration utilized.

Therapeutically effective amounts of the compositions described herein generally range from about 0.1 µg to about 25,000 µg (e.g., 1, 100, 500, 2000, 2500, 10,000, 15,000, 25,000 µg, 30,000 µg) of a complex of T helper epitope/dendrimer conjugated to a 7 µg to 3500 mg for a 70 kg patient (e.g., 0.1 µg of drug and 0.7 µg of the T-helper-dendrimer). In an embodiment in which the complex of T helper epitope/dendrimer is conjugated to AmBisome® for treating a *Leishmania* infection in a subject such as a mammal, the concentration of AmBisome® per dose is in the range of about 0.1 mg/kg to about 50 mg/kg. As is well known in the medical and veterinary arts, dosage for any one subject depends on many factors, including the subject's size, body surface area, age, the particular composition to be administered, time and route of administration, general health, and other drugs being administered concurrently.

### EXAMPLES

The present invention is further illustrated by the following specific examples. The examples are provided for illustration only.

### Example 1 - Immuno-potentiating Nanocarrier System Homing to Phagocytes Reduces AmBisome® Drug Dose

Since *Leishmania* species are obligate parasites in phagocytes, selectively delivering AmB to infected phagocytes improves efficacy, decreases systemic toxicity, and simplifies dosing regimens. To target AmB to phagocytes, we developed a peptide-derivatized dendrimer-AmB (PDD-AmB) nanocarrier platform by functionalizing dendrimers that complex with AmB with a peptide that targets MHC class II receptors. Since MHC class II receptor expression increases during the course of leishmaniasis, infected cells are desired targets for PDD-AmB. In a murine model for *Leishmania major* infection, PDD-AmB efficiently targeted infected phagocytic cells reducing the AmB effective dose by 80% with improved pharmacokinetics. Moreover, since the MHC class II targeting peptide is a universal helper T-cell epitope, PDD-AmB complexes elicited parasite specific T-cell responses. Thus, by reducing and simplifying AmB dosing and consequent side effects, and stimulating T-cell responses, PDD-AmB complex is a potential candidate for further development and human clinical trials.

Parasite uptake requires MHC class I and II molecules, and has also been associated with activation manifested in the up-regulation of MHC Class I and in particular MHC class II molecules. Universal T helper epitopes bind the flank of MHC class II of mice, rats, non-human primates and almost all humans. We have conceived a novel use of universal T helper epitopes to target (primarily) macrophages/ dendritic cells and monocytes (P. Daftarian, A.E. Kaifer, W. Li, B.B. Blomberg, D. Frasca, F., Roth F, Chowdhury R, Berg EA, Fishman JB, A1 Sayegh HA, Blackwelder P, Inverardi L, Perez VL, Lemmon V, Serafini P. Peptide-conjugated PAMAM dendrimer as a universal DNA vaccine platform to target antigen-presenting cells. Cancer Res, 71 (2011), pp. 7452-7462; Daftarian et al., Journal of Infectious Diseases, 2013. 208(11): p. 1914-1922; Daftarian et al., APC Targeted (DNA) Vaccine Delivery Platforms: Nanoparticle Aided IN "Molecular Vaccines Volume 2; From Prophylaxis to Therapy by Giese". Springer 2014). A nanocarrier decorated with universal T helper epitopes was designed that could complex with negatively charged AmB. Dendrimers are highly branched macromolecules which span from a central core and contain a series of structurally and synthetically distinct layers. Each layer added to the structure of a dendrimer is referred to as a "generation", in a clear reference to the stepwise growth of the macromolecule. PAMAM dendrimers have been developed and are now commercially available. The nanocarrier used in this report is a generation five dendrimer coupled to either Pan DR T helper epitopes or PADRE (PADRE-dendrimer hereafter referred to as PDD2), or to a T helper epitope of influenza virus haemagglutinin (HA) molecule (PDD1). The PDD1 or PDD2 provide advantages including reduction of off-targeting biodistribution and lowering the effective drug dose via selective targeting. In addition, intrinsic adjuvant activity of PDD drug delivery provides "help" for eliciting adoptive immunity in a therapeutic setting in the presence of antigens; converting infection to a built-in vaccine. It is known that CD4+ T helper cells play a vital role in the generation of CD8+ T-cell as well as humoral immune responses. Cytokines work at very low levels in a microenvironment, and have to be in the proximity of immune cells when antigen exposure occurs. The PDD is designed to make a complex with and to escort AmB to parasite reservoir cells, phagocytes, while they have an intrinsic/potent CD4+ T helper universal peptide to bring "HELP" for the host adoptive immunity. The *Leishmania major* antigens and the T helper will be expressed in the same APC resulting in enhanced specific T cell responses. Targeted delivery and encapsulation of amphotericin B in a biocompatible carrier is expected to reduce off-target effects, toxicity, and the effective dose while increasing stability. The targeted delivery of AmB to phagocytes requires a lower administered dose, reducing adverse drug reactions, and should results in a more efficient response. In addition, in the presence of infection, the intrinsic adjuvant activity of PDD results in mounting an adaptive immune response and to act as a built-in vaccine.

### Materials and Methods

Mice and inoculation of *L. major.* Female BALB/c mice, 8-9 weeks old, were used in this study. The metacyclic promatigotes of *L. major* (clone VI (MHOM/IL/80/Friedlin) were prepared as follows. Isolated parasites were washed twice in 20 ml of PBS and 0.5 micro liters of antibiotic solution for 20 min then cultured in a medium of 20% FBS and Schneider's media for 6-7 days. The parasites are washed twice with PBS prior to infection. For *in vivo* experiments, 1× 10⁶ metacyclic promastigotes were injected intradermally in the dorsum one inch in front of the base of the tail of mice. Inoculations consisted of metacyclic promastigotes suspended in 0.1 ml of PBS. The treatment was started once the lesions were between 20 and 70 mm² and was administered once a day for 10 days intraperitoneally. The positive control was AmBisome® administered at 37.5 mg/kg/day for 10 days. The animals were examined daily and clinical signs of disease were noted. Once a lesion formed, its size was monitored by measuring its two widest diameters at weekly intervals for 4 weeks. The lesion sizes were compared to mice infected but not treated. Moribund or sick animals were euthanized.

Peptides. The following peptides were used: Pan DR Epitope (PADRE): a(or A)KXVAAWTLKAAa(or A)ZC (SEQ ID NO: 33) (21stcentury); modified Hemoagglutinin HA110-120: SFERFEIFPKEC (SEQ ID NO: 34) (HA) (21stCentury). A third peptide was made of randomly scrambled amino acids and was used as a control nanoparticle.

Conjugation of the peptides to the dendrimers and characterization of the products. Peptide-dendrimer conjugates were manufactured by cross-linking 5th generation dendrimer (Dendritech, MI) and peptides target: PADRE and HA110-120 with an added cysteine at the c-terminus using maleimido-bis-succinimidyl ester (MBS) (Sigma-Aldrich, St. Louis, MO) as previously reported (Daftarian et al., Cancer Res http://cancerres.aacrjournals.org/content/early/2011/12/07/0008-5472.CAN-11-1766.full.pdf.). Briefly, capped, aminus terminus acetylated PADRE or HA peptide is covalently attached to the dendrimer by amide formation between one of the dendrimer primary amine (-NH₂) surface groups and the unprotected carboxylic acid (-COOH) terminus of the peptide. Reverse Phase-HPLC was used to purify HA-dendrimer, PADRE-dendrimer, and scrambled control peptide-dendrimer (G5). The reaction products were characterized using MALDI-TOF mass spectrometry, UV-Visible and ¹H NMR spectroscopy. The Peptide-dendrimer conjugates were characterized by MALDI-TOF Mass-spectroscopy (Matrix- assisted laser desorption/ionization) and ¹HNMR spectroscopic techniques. For MALDI-TOF Mass spectroscopy, the samples were dissolved in PBS buffer (Daftarian et al., Cancer Res. http://cancerres.aacrjournals.org/content/early/2011/12/07/0008-5472.CAN-11-1766.full.pdf).

Nanoparticle/AmB complexation. An AmB vial contains a calculated 50 mg of amphotericin B, which is reconstituted in 12 ml PBS for administration. The membrane also contains approximately 213 mg hydrogenated soy phosphatidylcholine; 52 mg cholesterol, 84 mg distearoylphosphatidylglycerol, 0.64 mg alpha tocopherol, 900 mg sucrose, and buffered with 26.25 mg disodium succinate hexahydrate. Prior to use, a vial of AmB was reconstituted with 13.5 ml sterile water resulting in a pH of 5-6. For making complexes, AmB was resuspended in warm (37°C) PBS (pH 7.4) at 3.6 mg/ml (Invitrogen). For AmB complexes with PDD1 or PDD preparations, AmB solution was added drop-wise to the nanoparticles using a p100 tip to an equal volume of nanoparticles while the vial containing nanoparticle was vortexed. Amine: Phosphate (N: P) ratios including 1:1, 2:1, 3:1, 5:1, and 7:1 were used unless otherwise indicated. In some cases for delivery analysis, a labeled payload control was used including dsRNA-Alexa Fluor 555 (hereafter referred as to AL4) (Invitrogen) or albumin-FITC (Sigma). The labeled control cargos (dsRNA or albumin) were used with nanocarrier (PDD1 or PDD2) or in a mixture with the AmB complex at a ratio of 0.1. The mixture of AmB/labeled control cargo was then added drop-wise to the nanoparticle solution. These preparations were analyzed after 20 minutes incubation at room temperature. The full dose of the AmB is 37.5/Kg/day for 10 days. To make one fifth of this dose complexed with AmB at a ratio of 1:7 (AmB:PDD1), 133ug AmB and 931 ug PDD1 were mixed.

Dynamic Light Scattering (DLS) analysis. Nanoparticles were detected using a Malvern Zetasizer Nanaoseries dynamic light scattering/molecular sizing instrument with an argon laser (Nano- ZS90). The instrument was operated with general-purpose resolution. Liposomes analyzed were fairly poly-dispersed in nature. Table 2 shows the average diameter of individual particles and complexes. All the samples were used as obtained without further dilution. For size determination, water was selected as a dispersant. All the samples were allowed to equilibrate for 5 sec before data acquisition. For each sample a single measurement with 20 acquisitions were carried out.

**Table 2 - Individual Particle and Complex Average Diameters.**

| Particle | Average diameter (nm) |
|---|---|
| PDD | N/D |
| AmBisome® | 114.5 |
| AmBisome®/PDD | 166 |
| AmBisome®/PDD/siRNA | 251.35 |

| | |
|---|---|
| Nanoparticle average sizes were analyzed with DLS. Particles were prepared as described in the material and methods section and were analyzed with DLS to determine average sizes. All the samples used were as obtained without further dilution. For size determination, water was the dispersant. All samples were allowed to equilibrate for 5 sec before data acquisition. For each sample a single measurement with 20 acquisitions was performed. | |

AmB complexed with either PDD1 or PDD2 with and without AL4 were prepared as described above in water and PBS. The sizes were analyzed after 20 min incubation at room temperature using the Malvern Zetasizer Nanaoseries dynamic light scattering/molecular sizing instrument with argon laser wavelength λ=830 nm, a detector angle 90. Light scattering experiments were conducted using at least 20 independent readings 10 second in duration. The plotted data represents an average of 6 DLS runs.

Assessment of binding and cellular internalization. Fluorophore labeled dsRNA (control double stranded RNA-Alexa Fluor 555 labeled or AL4), FITC labeled albumin, and complexes of nanoparticles (PDD1 or PDD2) with AL4 at specified ratios, were incubated for either 30 minutes or 2 hours with murine peritoneal macrophages, murine splenocytes, or human purified monocytes. Flow cytometry experiments were conducted to analyze the binding of nanoparticle/payload complexes to cells stained for the expressing MHC class II, macrophage, dendritic cells, or monocytes markers. A confocal microscope was used to examine the fluorescence distribution by z-sections. In another series of experiments, a GFP-harboring plasmid at 20 µg in 100 µl, free or complexed with PDD2 (P: N ratio of 1:7), was injected (s.c.) in the left flank of C56BL mice. The draining lymph nodes as well as the lymph node of the opposite flank were assessed by flow cytometry. The expression of GFP in DC was analyzed in the PDD2/GFP-plasmid and control groups. Viability of macrophages were assessed using Trypan Blue and annexin v-APC and found to be in the range of %13-24 for PDD2/AmB/AL4 and 8-13 % for media alone.

IFN-γ ELISA. We have recently (Daftarian et al., Cancer Res http://cancerres.aacrjournals.org/content/early/2011/12/07/0008-5472.CAN-11-1766.full.pdf.) used a similar PADRE-dendrimer platform for the efficient transfection of human and mouse B cells with nucleic acid. This platform/ method allow *in vitro* transfection of antigen presenting cells. The method features the use of PADRE-derivatized-dendrimers which specifically bind to MHC class II molecules expressed on APC to deliver *L. major* specific epitopes to T cells. As a result, PADRE-dendrimer/ DNA plasmids harboring *L. major* antigens transfect and make the splenocytes of the immunized mice display (present) epitopes in 48 hours. If the mice have elicited specific T cells, they will be stimulated to produce IFN-γ. The method does not substitute for the current techniques to measure T cell response but rather, it offers the possibility to selectively transfect the APCs in the PBMCs allowing the MHC unrestricted stimulation of the T cells. By using the whole repertoire of DNA present in *L. major* as source of antigens, this method allows the detection of immune reaction against known and unknown epitopes. Secreted IFN-γ upon stimulation by PDD/DNA harboring *L. major* antigens was measured using ELISA reagents from PharMingen (San Diego, CA). The control plasmid (Invitrogen™ Life Technologies) and PVAX with inserted genes KMPII, TRYP, LACK and PAPLE (Lang et al., J Cell Sci 107 (pt 1):69-82, 1994). PADRE-dendrimer was mixed with the harboring antigen and the control plasmids were left at room temperature for 20 minutes to form the complex. Splenocytes were co-cultured with PADRE-dendrimer/DNA complexes and IFN-γ ELISA was performed on the supernatants of the cultures 48 hours post-stimulation.

Transmission Electron Microscopy (TEM). Preparations were made of the nanoparticle, the nanoparticle and AmB, and the complex added to the population of cells. Each was preserved in a 2% formaldehyde solution and diluted 1:10 in distilled water. A pipette drop of each sample was placed on a carbon and nitrocellulose coated 200 mesh copper grid and allowed to air dry. The grids were then examined at several magnifications up to 245,000X in a Philips CM-10 TEM. Images were taken of each preparation using a Gatan digital camera.

Elicitation and Collection of Peritoneal Macrophages. Peritoneal macrophages were elicited as follows: 8 week old wild type BALB/c female mice were given 1.5 mL of sterile 3% thioglycollate via i.p. and sacrificed by CO₂ at day 4 of injection. AMB +/- PDD treatment was administered to these mice via i.p. during the last 2 hr of the 4 day elicitation period. Mice were anatomically positioned for dissection; a small incision was made in the skin of the lower abdomen to insert scissors to cut the abdominal skin throughout the midline of the body. The peritoneum was exposed and gently massaged. The portion of it corresponding to the upper abdomen was carefully lifted with a hemostat and the peritoneal cavity was irrigated with 5 mL of cold PBS utilizing a 5 mL syringe with a 26 ½ gage needle. Peritoneal exudates were collected by suction, carefully inserting a sterile Pasteur pipette through the top lateral aspect of the peritoneum. Exudates were place on ice for the remainder of the procedure. Collected exudates were washed in cold PBS and centrifuged for 10 min at 1200 rpm twice and viability assessed in the hemocytometer by trypan blue exclusion.

Statistical analysis. Sigma-Plot (Systat Software Inc. San Jose, Ca) or Prism statistical analysis (ANOVA; Prism software, GraphPad, San Diego, CA) was used for statistical analysis. One-way Analysis of Variance between groups (ANOVA) was performed after normality evaluation by the Kolmogorov-Smirnov test. Pairwise post-hoc analysis was performed using the Holm-Sidak's test or the Dunn's test. ANOVA on Ranks was used if the sample was not normally distributed. The Student's T-test was used to compare two groups. Kaplan Meier log-rank followed by the Holm-Sidak *post-hoc* analysis was used to evaluate differences in survival between groups.

### Results

We have developed a novel biodegradable targeted nanoparticle platform for drug delivery into cells of the mononuclear phagocyte system to reduce drug dose/toxicity and enhance immune response involvement in controlling the infection. The nanocarrier platform is composed of a drug binding PAMAM dendrimer which is decorated with an MHC class II binding ligand peptide (universal pan DR binding peptide) with intrinsic immunopotentiating activity. The dendrimer moiety (positively charged) complexes with AmB (negatively charged). Our group and others have shown that the universal T-helper peptides bind specifically to MHC class II molecules of mice, non-human primates and humans. In addition, these universal T-helper epitopes have the capacity to activate DC and macrophages, inducing immune responses, thus making the platform an ideal candidate for both drug delivery and anti-*Leishmania* immune activation. The PDD1 and PDD2 synthesis were performed and characterized as previously described (Daftarian et al., Cancer Res http://cancerres.aacrjournals.org/content/early/2011/12/07/0008-5472.CAN-11-1766.full.pdf) and below. PDD1 was designed specifically for the experiments performed on BABL/c while PDD2 uses PADRE a universal helper T cell epitope that works on mice and human.

Characterizations of peptide-dendrimer and the PDD/drug (payload) complexes. To assess the targeting ability of AmB, albumin-FITC and dsRNA-Aexa Flour 555 (AL4) were used as cargo. MALDI-TOF, proton NMR, DLS, and TEM were used to characterize the dendrimer-peptide conjugates and complexes at different ratios of the PDD and AmB, or PDD control Alexa Fluor coupled dsRNA (AL4). Peptide substitutions or the average number of peptides coupled to dendrimer was assessed via MALDI-TOF mass spectrometric experiments. Each newly synthesized batch was compared with a previously preparation to assure the consistency of products by chemical characterization. As shown in Figure 12, the average targeted peptide numbers on each nanoparticle was 2. This design would guarantee that a majority of the amine groups on the product surface were maintained resulting in a strong positive charge on surface modified nanoparticles under physiological pH conditions. The difference in mass between PDD and G5 dendrimer was determined. While dendrimer mass is 26184.2, the MW of PDD is 3295 and 3187 in batches #1 and #2 respectively. The analysis indicates that there is an average of 2 PADRE peptides (MW 1613) linked to each dendrimer molecule surface. ¹HNMR spectra of all the three samples show that sample 2 and sample 3 are very similar in the structure (Figure 12). To verify that PDD was able to complex with AmB, both DLS and TEM studies were performed. Both PDD1 and PDD2 were mixed with AmB in a 10:1 weight ratio in PBS. After a 20 minute incubation time at room temperature, the complexes were analyzed using DLS and TEM, which demonstrated complexation of AmB and PDD.

TEM and DLS Analysis. PDD showed a low polydisparity index in TEM assessment that was confirmed in multiple DLS measurements. AmB exhibits a particle size distribution (∼110 nm, Figure 3) which correlates very well with the DLS measurements (114.5 nm). TEM observations indicate that PDD had approximately 10 nm in size. There was no detectable size difference between PDD1 and PDD2. TEM observations also indicated that AmB encapsulating PDD (PDD/AmB) displays a high degree of poly-disparity as confirmed by our DLS studies. The AmB size varied from 50 nm to 290 nm. Small (60 nm -90 nm) light colored AMB (3.7 % of the total population), exhibits a low loading of PDD (Figure 3). In addition, dark, large (∼ 150nm) AMB appears to indicate high loadings of PDD2 within the AMB nanoparticles (Figure 3) constituting 22% of the total nanoparticle population. The TEM observations confirm the DLS data (166 nm) for AMB/PDD. In addition, we also observed that approximately 11% of the PDD/AmB (AmB coated with nanoparticles) were approximately 166 nm in size and an additional 11% were approximately 200 nm size. This change in AmB nanoparticle size from ∼114 nm to ∼166 nm and 200 nm after encapsulation of PDD corresponds to a 45.6% and 75% change in size respectively. In addition, the dark particles observed in the TEM may indicate remaining PDD not encapsulated by the AmB or PDD excluded during bursting of the AmB. In some cases, we observed dark and very large PDD/AmB (200nm to 285 nm). However, these constituted only 7.4% of the total AmB population. From the TEM observations it is evident that more than 95% of the AmB nanoaprticles have encapsulated PDD. Nevertheless, other than MALDI-TOF, the complexation of PDD and PDD1 with AmB was clearly shown in TEM and DLS (Figure 3 and Table 2). DLS analysis showed a size increase in the particles when AmB was complexed with PDD, and an additional increase in the diameter of the particles when PDD was complexed with labeled AL4 (Table 2). The Zetasizer size detection range is below the size of the PDD alone so it was not detected. The average diameters of AmBisome®, PD/AmB, and PDD/AmB/AL4 were 112 nm, 154 nm, 254 nm respectively.

Platform targets macrophage/monocytes and spleens *in vitro* and *in vivo.* To examine the macrophage targeting ability of the platform, we initially used a negatively charged cargo, AL4, which is a dsRNA labeled with Alexa Fluor 555. Peritoneal-enriched macrophages from BALB/c orC57BL mice were isolated and incubated with AL4 with or without PDD1. A 20-minute incubation was sufficient for the complex of PDD1/AL4 to enter the macrophages. After incubation, cells were washed twice and images were taken using a Leica fluorescence microscope. An overlay of bright field and red fluorescent images demonstrates the ability of dendrimer to deliver AL4 to target murine macrophages (Figure 4A). Similarly, when purified human monocytes were co-cultured with PDD/ dsRNA-FITC, greater than 90% of human monocytes were positive for FITC (Fig. 4B). Human monocytes are generally difficult cells to transfect. Culture with dsRNA-FITC alone resulted in less than 1% transfection (cargo uptake). PDD/AL4 or scrambled-peptide-dendrimer/AL4 was then complexed and prepared for injected subcutaneously (s.c.) in the right flank of mice. One hour after s.c. injection of the PDD/AL4 and control complex, mice were euthanized, the spleens were harvested for IVIS imaging. The spleen from C57B1/6 mice injected with PDD/AL4 were positive (Figure 4C, emission of red fluorescent related to presence of AL4), while the control complex (AL4 complexed with a dendrimer coupled with a control random scrambled peptide) was negative. These data suggests that the PDD and PDD1 platforms complexed with a cargo actively target phagocytes, *in vitro and in vivo.*

Platform shuttles the cargo into macrophage and DCs *in vivo* and *in vitro.* The first design challenge in developing a targeted drug delivery nanosystem is to discover the correct ligand on the cell membrane that is more or less specific to the targeted cell lineage(s). Targeting a phagocytic system that includes different cell types is complicated. The use of a MHC class II molecule that is expressed in monocytes, macrophages, and DC is a feasible solution. However, the nano-vehicle should not only be able to penetrate/bind to its intended target cells, it must also transport the payload inside the cells. PDD complexed with GFP DNA was injected subcutaneously (s.c.) into C57BL mice. Five days post injection the draining lymph nodes were harvested and analyzed. Controls included mice injected with PBS or with a pMAX-GFP complexed with control dendrimer. FACS analysis (Figure 5A) revealed a significantly higher number of GFP+ cells, MHC class II+ cells and GFP+ DC+ in the draining lymph node of mice treated with PDD/pMAX-GFP. Similarly, confocal microscopy studies showed that when complexed with PDD, FITC labeled albumin was shuttled into purified macrophages (Figure 5B). Furthermore, PDD/GFP-DNA may be difficult to transfect human B cells (Figure 13A) and PBMC from non-human primates also were transfected with PDD/AL4. These data demonstrate that the platform is able to effectively shuttle a payload into phagocytic cells. In addition, the *in vivo* expression of GFP in DCs of draining lymph nodes demonstrate that the platform can complex, escort and deliver the cargo into dendritic cells in the host.

Platform targets AmB to macrophages, *in vitro* and *in vivo.* Since both AL4 (red tagged dsRNA) and AmB are negatively charged, they complex with the positively charged PDD. The confocal microscopy and flow cytometry studies using purified macrophages and various ratios of PDD/AL4/AmB indicated that complexes of [PDD:AL4:AmB] made of 7:5:1 weight ratios were able to transfect efficiently (Figures 7A and B).The addition of AL4 to the PDD/AmB complex enabled us to perform cell entry studies via monitoring/ visualizing AL4. We examined the uptake of the PDD/AmB/AL4 complex by macrophages *in vitro* and *in vivo.* For the *in vitro* studies, the complex was co-cultured with macrophages followed by flow cytometry analysis at several time points. The elicitation and collection of peritoneal macrophages were performed as described in the materials and methods and macrophage enriched cells were obtained (Fig. 6). This macrophage enriched population was used in the *in vitro* studies on the AmB targeted delivery by PDD. AmB was complexed with either [dendrimer/ AL4, as a control], or PDD/ AL4. The complexes were co-cultured for 10 or 120 minutes and cells subsequently stained with a macrophage marker F4/80 and assayed by flow cytometry (Fig. 7A). An average of a 17-fold increase in cells positive for PDD/AmB/AL4 was observed when compared with dendrimer-AmB-AL4. PDD significantly enhanced the drug targeting by peritoneal macrophages *in vitro* (Figure 7A).

To examine the effect of PDD on *in vivo* AmB delivery, AmB was complexed with control dendrimer and AL4 or with PDD and AL4 (7:5:1 weight ratios). The complexes were i.p. injected 2 hours prior to collection of cells from the peritoneal cavity. PDD enhances the *in vivo* AmB uptake in the peritoneal macrophages by ∼ 6-fold when cells were removed 2 hours post i.p. injection (Figure 7B). A chart summarizes the average experimental increase in the AmB uptake (Figure 7C). Both PDD and PDD1 exhibited similar results as shown in the data from representative experiments.

Determination of the effective AmB dose in a therapeutic *L. major* cutaneous BALB/c setting. To determine the tolerated effective dose of AmB that results in reduction of lesions, one million parasites were inoculated in the base of the tails of mice. Different doses were tested for the efficacy of AmB on lesion growth. The results of multiple experiments performed on optimized therapeutic cutaneous *L. major* model in our laboratory indicated that doses less than 37.5 mg/kg/day, for 10 days had only modest effects in lowering the lesion area. However, a dose of 37.5 mg/kg/day for 10 days resulted in significant decrease in the lesion area (Figure 8). Doses of 6.2 mg, 12.5 mg, or 25 mg (per kilogram, per day for 10 days) did not result in reduction of lesion sizes, but had modest effects in that this dose apparently slowed down lesion formation as compared to the group of mice that received no treatment (Figure 8). A dose of 50 mg/kg/day resulted in 21% weight loss. Therefore, an ineffective low dose of 6.2 mg/Kg/day (for 10 days) was chosen with or without the PDD1 or PDD.

Effectiveness of AmB increases 80% when wrapped in PDD1. The final determination of the efficacy of a targeted drug delivery platform comes from *in vivo* testing in a stringent/physiologically relevant animal model. *L. major* causing cutaneous leishmaniasis in BALB/c mice was accepted as a model resembling natural injection. We used an aggressive setting where treatment started when the lesions reached an average size of 20-70 mm². In this optimized therapeutic setting, the effective dose of 37.5 mg/kg/day for 10 days controlled the infection in a majority of mice. We performed two sets of experiments in mice in groups of 10 in which the lesion sizes were compared in groups receiving the complete dose, a dose 5 times lower (6.25 mg/kg/day for 10 days), and control groups. As is described in the materials and methods, BALB/c mice were inoculated (s.c.) with *L. major* parasites and 17 days post parasite inoculation the therapies began (when the lesions reached an average size of 20-40 mm²). All drug combinations were administered intraperitoneally. The percentage of change in the lesion sizes was measured. The low dose AmB (6.25 mg/Kg/day, for 10 days) does not reduce the lesion sizes while AmB/DRHA (AmB in complex: 6.25 mg/Kg/day, for 10 days) significantly reduces the lesion sizes. This data shows a benefit in utilization of the nanoparticle, as it clearly enhances the efficacy of AmB as compared with the un-capsulated drug. This treatment was not associated with any weight loss, which was observed in doses higher than 37.5 mg/K/day for 10 days. The rapidity of the efficacy of low dose AmB when bound to PDD1 (AmB at 6.2 mg/Kg/day, for 10 days) is superior to that of 37.5 mg/kg/day for 10 days in controlling lesions (Figure 9). Interestingly, the effect of reduction in lesion sizes in the group receiving AmB/DRHA appears much earlier than that of the complete dose, consistent with the immunopotentiating effect of the platform in involving an immune response. As early as day 7 post therapy, PDD/AmB treatment resulted in an effect carrying AmB at a dose of 1/5 the effective dose (Figure 9). This rapid acting/kinetics of AmB/PDD1 (6.25 mg) was observed in mice bearing active infection, on the day 53 post infection and only 7 days post-treatment, while 21 days was required for the full dose (37.5 mg AmB without PDD1) to result in similar effects.

The overall effects of various treatments in mice bearing skin leishmaniasis lesions ranging from 20-70 mm are shown in Figure 10. Lesions measurements are shown on days 0, 12, and 21 post therapies; the AmB at low (AmB70) and high doses (AmB370), AmB low dose wrapped in PDD1 (AmB70/PDD1), and untreated are shown. The IACUC protocol requires that the mice are sacrificed when the lesion size reaches more than 150 mm² or 60 days post parasite inoculation. Mice which received the AmB/PDD1 7mg dose are comparable with those which received 37.5 mg dose regime (Figure 10). Also shown is that the lesions in mice which received AmB/PDD1 7mg were significantly lower (P value = 0,0034) than those who received AmB 6.25 mg treatment. Also demonstrated is that the effect of AmB complexed in PDD1 is exhibited much sooner than the higher dose regimen (Figure 10).

PDD1/AmB and PDD/AmB elicit antigen specific immune responses. Day 60 post-parasite inoculation and 21 day post initiation of the therapy, splenocytes of mice were stimulated with *L. major* antigens as is described in the material and methods. Increases in antigen-specific IFNγ productions were detected in mice that received PDD1 or PDD2/AmB or PDD2/AmB as compared to mice that received AmB alone or AmB with the control nanoparticle (random-scrambled peptide-dendrimer). The splenocytes of mice treated with PDD/AmB after 48 hours incubation with plasmids harboring four *L. major* antigens expressed large amounts of IFNγ compared to splenocytes from groups treated with AmB alone, AmB complexed with control nanoparticle or PDD alone (Figure 11). This antigen-specific IFNγ production is important, since the combination therapy with a chemotherapeutic and an immunoenhancing agent (CD4+ T helper epitope) can convert the infection to a vaccine-inducing immune response by educating immune responses of the host (e.g., subject being treated). Consistent with many reports on the transient lymphoadenopathy and splenomegaly upon using immunopotentiating agents, we too observe a transient splenomegaly in mice receiving 10 doses of AmB/PDD. It was observed that spleen sizes were enlarged in the group receiving PDD1 (Figure 11B) which is also reported in the case of potent adjuvant such as CPG treatment. This is considered a sign of expansion of immune cells and the involvement of the immune system. Indeed, an antigen-specific T cell response was only mounted in mice that received PDD/AmB treatment and not in any other treatments (Figure 11C). Figures 11A and B show that the draining lymph nodes and spleens from C57BL or BALB/c mice that received i.p. injections of PDD1/AmB or PDD2/AmB (accordingly) were significantly enlarged on the day 5 post injections whereas the Scramble (Random)-peptide-dendrimer complexed with AmB did not show any such effect. A transient enlargement of the draining lymph nodes and spleens of mice receiving PPD/AmB is clearly shown. Figure 11C shows the result of antigen-specific IFNγ production. Mice in groups of five received either no injections, PDD1/AmB or AmB with control dendrimer, splenocytes co-cultured with plasmids harboring *L. major* antigens complexed with PDD1. IFNγ was measured in the supernatant after 48 hours and the group that received PDD1 (or PDD2) complexed with AmB showed enhanced IFNγ production indicating elicitation of *L. major* specific T cell immunity. Panel D of the figure 11 shows the increased expression of MHC class II, a sign of activation of immune responses, in mice that received PDD/AmB and not in those that received the control dendrimer plus AmB.

Parasite burden was assessed in the spleens of the treated mice on day 21 posttreatments. QPCR detection of parasite in the DNA of the spleen of the mice treated with AmB/PDD or AmB alone demonstrated a significant decrease in the parasite copies (parasites in the 100mg of spleen) in groups of mice which received AmB/PDD versus the groups that received AmB alone (1900 parasites in the 100mg of spleen).

### Summary and Discussion

Amphotericin-B is an effective anti-leishmaniasis agent; however the use of this drug can result in serious toxicity including, but not limited, to kidney failure. Alternatively, AmBisome® (AmB, liposome wrapped amphotericin-B) exhibits reduced toxicity and improved efficacy for treatment of leishmaniasis in humans. However, these benefits over Amphotericin-B only occur at doses lower than 5 mg/kg/day. Indeed, AmB is administered intravenously with extra care at a daily dose of 1.0 mg/kg/day and increased stepwise to 5.0 mg/kg/day, as required. To reduce the efficacious dose required, a homing target is required to direct treatment to infected cells. *Leishmania* parasites infect phagocytes including monocytes, macrophages, and dendritic cells, cells that express MHC class II molecules. MHC class II expression increases during the course of leishmaniasis making infected cells a superior target for an MHC class II binding nanocarrier.

Since *Leishmania* species are obligate intracellular parasites of the afore mentioned phagocyte system, our principal aim was to create phagocyte targeting nano-carriers (Peptide-Dendrimer, PDD and HADD) platforms that could be coupled to a drug such as AmB for delivery into phagocytic cells using an MHC class II homing peptide. For this investigation, PADRE universal peptide was chosen as the targeting polypeptide based on its potential to confer various advantages, including further translation to human to the platform. For example: a) PADRE binds with high affinity the MHC class II complex H2^{b}, monkey MHC class II and 95% of human HLADR allowing an easy translation of this platform into the clinic; b) it is a non-self antigen and thus it is not recognized by thymus derived natural regulatory T cells but provides helper signal.

Generation of this nanocarrier system was accomplished by designing a biodegradable nanostructure platform decorated with a cell-specific homing peptide at a low substitution. The platform was tailored to target phagocytes via the homing peptide while facilitating cell entry escorting a payload (e.g., an anti-pathogen drug). Since phagocytes express MHC class II, we conceived of the use of MHC binding peptides that not only target macrophage, monocytes, and dendritic cells, but also activate CD4 T helper immune responses. This nano-carrier system has positively charged amine groups that make stable complexes with the negatively charged AmB and escorts the AmB directly into phagocytic cells *in vivo.* Two drug-carrying phagocyte binding platforms were created using two different MHC class II binding peptides. Dendrimer was conjugated to either PADRE: a(or A)KXVAAWTLKAAa(or A)ZC (SEQ ID NO: 33) resulting in dendrimer-PADRE (PDD2) or to a modified Hemoagglutinin HA110-120: SFERFEIFPKEC (SEQ ID NO: 34) resulting in a dendrimer-HA (PDD1).To examine the targeting capacity of platforms, both *BALB*/*c* and *C57BL*/*6* mice (H2b and H2d MHC alleles, respectively) were used. Since HA peptide binds specifically to MHC class II of BALB/c mice that are susceptible to *L. major,* this platform was initially selected for the therapeutic leishmaniasis studies. The therapeutic studies were initially conducted using this HA-dendrimer (PDD1) in mice bearing lesions greater than 20 mm². The PDD2 platform is decorated with PADRE, a promiscuous universal T helper epitope that binds to DC/phagocytes of a wider range of species including mice, in particular C57BL/6 (PADRE is known to have a better binding affinity to H2d than it does to H2b allele), non-human primates, and human via binding to their MHC class II molecule. Therefore, Peptide-Dendrimer (PDD) is an ideal candidate for translation to humans. Here, the ability of PDD to target macrophage, monocytes, and dendritic cells was evaluated in *in vivo* and *in vitro* in C57BL/6 mice, and *in vitro* in non human primates and humans. Both HA-dendrimer (PDD1) and PDD2 platforms were used to study the targeted deliveries in a cutaneous leishmaniasis model in susceptible mice, BALB/c. Both PDD1 and PDD2 target phagocyte, shuttle their cargo into these cells, activate CD4 T helper cells, and act as an adjuvant to enhance immune responses.

Since the affinity of PADRE for H2^{d} is not well established in the literature, we designed a dendrimer-peptide using the HA which binds with a high affinity to H2^{d} (BALB/c background). Using a murine model for *Leishmania major* infection, our results show that this novel nanoparticle platform is able to escort its cargo into phagocytic cells, including macrophages and dendritic cells. Efficacy in reducing lesion sizes at a dose five times lower than the standard effective dose was observed. The kinetics of treatment was also improved. Utilization of the PDD-AmB platform allows for lowering of the effective dose and frequency of drug administrations, concomitant with reduced AmB associated toxicity, making this platform a candidate for application in human clinical trials.

Previous findings have shown that immunopotentiating agents such as ligands of various toll like receptors (TLRs) may be used as prophylactic and/or therapeutics agents for various infectious diseases; examples are isatoribine and ANA975 (TLR-7), Class C CpG, IMO-2125 (TLR-9), MPL (TLR-4), Imiquimod (TLR-4), Resiquimod (TLR7/8), PolyI:polyC12U (TLR3), and E5564 or TAK-242 (TLR-4). These therapies however, act via activation of innate immune system components while the use of PDD complexes not only offers targeted drug delivery but also enhances adaptive immunity in an equally targeted manner. PDD drug-carrying nanoparticles indiscriminately target phagocytes in mice, non-human primates, rats, and humans making the platform an ideal candidate for translational studies involving generalized drug delivery to phagocytes. Herein we describe a novel use for such nanoparticle delivery systems; in addition to targeting MHC II+ phagocytic cells this nanocarrier concomitantly allows for stimulation of adaptive immune responses. In some embodiments, however, the PDD compositions and methods described herein may be combined with one or more of the ligands described herein (e.g., TLR-7, TLR-9, TLR-4, TLR7/8, TLR3, etc.) for treating a subject in need.

*Leishmania* parasites use mononuclear phagocytes as reservoirs. The AmB carrying nanovehicles described here (PDD and HADD) effectively targeted phagocytes and resulted in i) lowered AmB effective dose, ii) enhanced response kinetics, iii) lowered toxicity, and finally, iv) immunoenhancing effects that assist the host in mounting anti-*Leishmania* specific T cell responses.

In summary, *Leishmania* parasites use phagocytes as reservoirs. The AmB carrying nanovehicles described here (PDD) effectively target phagocytes and result in i) AmB lowered effective dose, ii) enhanced rapidity of the response, iii) lowered toxicity, and, iv) immunoenhancing effects that assist the host to mount anti-*Leishmania* T cell responses.

### Example 2 - Ocular Therapies

The compositions and methods described here may be used in therapeutic ocular applications. In ocular applications, the compositions may be delivered topically or intravitreally. Many if not all of the infections described herein have ocular involvement, and thus a local therapy using the methods and compositions described herein would be of enormous utility. Examples of ocular conditions that can be treated and/or prevented include Fungal Keratitis (specific for ampho) and other ocular-relevant infections related to intracellular organisms such as toxoplasmosis, river blindness (onchosarchiasis), CMV and herpes (e.g., HSV and HZO).

### Example 3 - Liposome-based drugs on the market and in clinical trials

Based on the ability of the dendrimer to bind specific classes of lipids in the liposome and the general use of only a few types of lipids in these formulations, all drugs either approved, or under development are encompassed. It is expected that drugs with similar lipid compositions as Ambisome should be bound and targeted. See Figures 14 and 15 which include Tables listing examples of some of the drugs which may be delivered using the compositions and methods described herein.

### Example 4 - A targeted and adjuvanted nanocarrier lowers the effective dose of liposomal Amphotericin B and enhances adaptive immunity in murine cutaneous leishmaniasis

Described herein is a novel use of a peptide-dendrimer nanocarrier that has been shown to target major histocompatibility complex class II (MHC II) expressed on human and mouse APC (e.g. macrophages, and dendritic cells) (Daftarian et al., Peptide-conjugated PAMAM dendrimer as a universal platform for antigen presenting cell targeting and effective DNA-based vaccinations, Cancer Research 2011). The platform uses a Pan-DR-binding epitope (PADRE) as its APC homing moiety. PADRE is a promiscuous CD4⁺ Th determinant that binds to most murine and human MHC II molecules, and has been previously used as an adjuvant to enhance immune system responses in many studies, including human trials. Targeting APCs is a novel use of universal T helper epitopes such as PADRE. PADRE-derivatized dendrimers (PDD) have resulted in effective targeting of DNA to APCs for gene-based vaccination. Since *Leishmania* are obligate intracellular parasites, T cell responses and Th1 cell-mediated immunity are required for control of the infection and CD4⁺ Th1 cells, in particular, are indispensable for resistance. Therefore, we reasoned that PDD, a nanocarrier that targets APC and elicits a Th1 response, should be able to carry LAmB to the source of infection (macrophages) while also enhancing Th1 adaptive immune responses.

The nanocarrier reported here is a generation 5 Polyamidoamine (PAMAM) dendrimer coupled to PADRE (PADRE-dendrimer or PDD1). We also designed an alternative T helper epitope-dendrimer platform specific for BALB/c mice where we conjugated a T helper epitope of influenza virus haemagglutinin (HA) molecule (PDD2). Both platforms are positively charged and can bind drugs with a negative net charge and protect them from degradation (Daftarian et al., Peptide-conjugated PAMAM dendrimer as a universal platform for antigen presenting cell targeting and effective DNA-based vaccinations, Cancer Research 2011; Medina SH, El-Sayed MEH. Dendrimers as carriers for delivery of chemotherapeutic agents. Chem Rev 2009; 109:3141-57). While PDD2 only binds to APCs bearing the H-2^{d} haplotype (BALB/c), PDD1 can bind to MHC II on APCs of both human and mice. Additionally, since CD4⁺ T cells play a vital role in the generation of CD8⁺ T cells as well as humoral immune responses, we hypothesized that the intrinsic adjuvant activity of PDD drug delivery may induce anti-leishmania adaptive immunity in a therapeutic setting where leishmanial antigens are produced at the site of infection (Figure 2). Therefore, in an infected host, PDD1 or PDD2 may convert infection to a therapeutic vaccine by inducing an immune response in APC at the site of infection.

### MATERIALS AND METHODS

### Conjugation of peptides to dendrimers and characterization of products

The following peptides (21st Century Biochemicals, Inc.) were used: Pan-HLA-DR-binding epitope (PADRE): a(or A)KXVAAWTLKAAa(or A)ZC (SEQ ID NO: 33); influenza Hemoagglutinin-derived HA110-120: SFERFEIFPKEC (SEQ ID NO: 34) (HA), which is a H2-I-Ed-restricted peptide that can be used in BALB/c. A third peptide, made of randomly scrambled amino acids, was used for a control nanoparticle (random peptide-control-dendrimer or ScrDR). Peptide-dendrimer conjugates were made and characterized as described before (Daftarian et al., Peptide-conjugated PAMAM dendrimer as a universal platform for antigen presenting cell targeting and effective DNA-based vaccinations, Cancer Research 2011; see Figure 12 and methods section).

### PDD/LAmB or PDD/LAmB-Rhodamin complexation

See FIG. 20.

### Dynamic Light Scattering (DLS) analysis.

Nanoparticles were detected using a Malvern Zetasizer Nanaoseries dynamic light scattering/molecular sizing instrument with an argon laser (Nano- ZS90). The instrument was operated with general-purpose resolution. For size determination, water was selected as a dispersant. All the samples were allowed to equilibrate for 5 sec before data acquisition. Single measurements with 20 acquisitions were carried out. LAmB complexed with either PDD1 or PDD2 was prepared in water or normal saline. Sizes were analyzed after 20 min incubation at room temperature using the Malvern Zetasizer Nanaoseries dynamic light scattering/molecular sizing instrument with argon laser wavelength λ=830 nm, detector angle 90°. Light scattering experiments were conducted using at least 20 independent readings 10 sec in duration. The plotted data represents an average of 6 DLS runs.

### Transmission Electron Microscopy (TEM)

Preparations were made of the nanoparticle, the nanoparticle and AmB, and the complex added to the population of cells. Each was preserved in a 2% formaldehyde solution and diluted 1:10 in distilled water. A drop of each sample was placed on a carbon and nitrocellulose coated 200 mesh copper grid and allowed to air dry. The grids were then examined at magnifications up to 245,000X in a Philips CM-10 TEM. Images were taken of each preparation using a Gatan digital camera.

### Cytotoxicity assay

Cytotoxicity was performed on Hep2 cells and evaluated using the MTT assay.

### Assessment of cell specific targeting and cellular internalization

In vivo macrophage uptake of LAmB-Rh and complexes of PDD (PDD1 or PDD2)/LAmB-Rh at specified ratios were also evaluated . After receiving i.p. injections of either of LAmB-Rh or PDD/LAmB-Rh, mice were euthanized and macrophages from the peritoneal cavity were collected as is described in the materials and methods. Murine peritoneal macrophages were either resident or induced as described. Flow cytometry and confocal florescent microscopy experiments were conducted to analyze the binding of complexes to cells. A confocal microscope was used to examine the fluorescence distribution by z-sections.

### Elicitation and collection of peritoneal macrophages

Peritoneal macrophages were elicited as follows: 8 week old wild type BALB/c or C57BL female mice were given 1.5 mL of sterile 3% thioglycollate via i.p. and sacrificed by CO₂ at day 4 of injection. LAmB-Rh +/- PDD or ScrDR treatments were administered to these mice via i.p. injection during the last 2 hrs of the 4 day elicitation period.

### Mice and inoculation of L. major

For *in vivo* experiments, 1×10⁶ metacyclic promastigotes were injected intradermally in the dorsum one inch in front of the base of the tail of mice. Treatment was started once the lesions were between 20-70 mm² in size and was administered once a day for 10 days intraperitoneally (i.p.). The positive control was LAmB administered at 37.5 mg/kg/day for 10 days. The animals were examined daily and clinical signs of disease were noted. Once a lesion formed, its size was monitored by measuring its two widest diameters at weekly intervals for 4 weeks. Lesion sizes were compared to mice infected but not treated. Moribund or sick animals were euthanized as described in the University of Miami IACUC approved protocol. All mouse studies were approved by the Institutional Animal Care and Use Committee at the University Of Miami Miller School Of Medicine, and all animals were housed in American Association for Accreditation of Laboratory Animal Care-approved facilities.

### IFN-γ ELISA

Owing to the ability of universal T helper to bind MHC class II, we have recently used a nanoparticle platform for targeted transfection of mouse APC cells with nucleic acid resulting in the expression of native form of the antigen. Here, the APC targeting moiety is an I-E^{d} MHC class II epitope (H-2-IEd), SFERFEIFPKEC (SEQ ID NO: 34), or HA, that specifically binds BALB/c MHC class II, expressed on BALB/c APCs. The HA-dendrimer (HADR) complexes DNA and escorts it into APC. This method creates a novel T cell immunomonitoring platform that features the use of HADR/DNA (e.g. plasmid DNA encoding for *L major* antigens) to generate APC expressing *L. major* specific epitopes for presentation to T cells, all in the same well. Therefore, to assess the antigen specific T cell responses in treated mice, we employed HADR complexed with a mixture of four DNA plasmids harboring *L. major* antigens (KMPII, TRYP, LACK and PAPLE22).

Mice, 5 per group, with *L. major* lesions received i.p. injections of PDD/LAmB (low-dose), Scr DR/LAmB (low-dose), or L-AmB (full-dose) for 10 days. Splenocytes from individual mice was isolated, washed with RPMI 1640 culture medium supplemented with 10% FCS and penicillin, and placed at 1 × 10⁶ cells/well in 96-well tissue culture plates. Splenocytes from treated animals were stimulated with HADR (40 µg) complexed with a set of plasmids (4 µg) that encode for *L. major* antigens. HADR (40 µg) complexed with empty pVAV (4 µg) was used as a "background" in parallel wells for each test. IFNγ measurements in the splenocyte culture supernatants were performed using an ELISA kit (R&D Systems, MN) after plates were incubated at 37°C 5% CO₂ for 48 hours. IFNγ "background" measurements were subtracted from all data. Secreted IFN-γ upon stimulation with HADR/ L major plasmids was measured using ELISA reagents. The control plasmid (Invitrogen Life Technologies) and pVAX with inserted L major 4 antigens genes (provided by Professor Jordi Alberola Domingo, LeishLAB Universitat Autònoma de Barcelona Spain) were used in this study. In brief, after 40 µg of HADR was mixed with the four L major encoding plasmids (total of 4 µg, 1 µg of each plasmid) or with the control plasmid (empty vector, 4 µg), the tubes were left at room temperature for 20 min to form the complex. All HADR/plasmid complexes were made in saline. Splenocytes were co-cultured with HADR/DNA complexes and IFN-γ ELISA was performed on the supernatants of the cultures 48 hrs post-stimulation.

### Real-time PCR amplification

*Leishmania* DNA in tissue samples was detected by real-time PCR. Briefly, the forward primer, reverse primer and the Taq-Man MGB probe were designed to target conserved DNA regions of the kinetoplast minicircle DNA from *Leishmania sp.* Eukaryotic 18SRNAPre-Developed TaqMan Assay Reagents (Applied Biosystems, Foster City, CA) were used as an internal reference. Each amplification was performed in triplicate in a 25 µL reaction volume (TaqMan Universal PCR Master Mix; Applied Biosystems). The thermal cycling profile was 50 °C for 2 min, 95 °C for 10 min, 40 cycles at 95 °C for 15 s, followed by 60 °C for 1 min. *Leishmania* DNA load in each sample was determined by relative quantification using the 2-ΔΔCt method. Results are expressed as x-fold more DNA copies than the calibrator sample. The calibrator was a spiked sample with a known number of parasites.

### Statistical analysis

One-way Analysis of Variance between groups (ANOVA) was performed after normality evaluation by the Kolmogorov-Smirnov test. Pairwise post-hoc analysis was performed using the Holm-Sidak's test or the Dunn's test. ANOVA on Ranks was used if the sample was not normally distributed. The Student's T-test was used to compare two groups. Kaplan Meier log-rank followed by the Holm-Sidak *post-hoc* analysis was used to evaluate differences in survival between groups.

### RESULTS

### PDD/LAmB complex characterizations

PDD, positively charged, and LAmB, negatively charged, complex readily in solution. PDD-LAmB complex formation was confirmed using TEM and DLS analysis (Fig. 16A and B). The size of the PDD, LAmB and PDD/LAmB complexes were analyzed by TEM and DLS. It was observed that the PDD sample was ∼ 11.7 nm (99.9%) in diameter while LAmB to be 124.2 nm in size. The electrostatic assembly of the two resulted in the PDD-LAmB complex of size 141.8 nm. The expected size of the complex (∼ 147.6 nm) and the DLS data matched closely confirming stable PDD/LAmB complex formation. Similarly, results obtained by DLS are corroborated by TEM micrographs. A ratio of 1:10 (LAmB:PDD) was used in these studies based on our previous work. This ratio has resulted in efficient shuttling of the cargo and is associated with strong non-toxic immunopotentiating activity. The TEM clearly shows that LAmB is decorated with multiple PDD molecules (Fig. 16A).

### Non-toxic in vivo macrophage targeting

Next, LAmB was labeled with Rhodamin B, a positively charged fluorochrome (Fig. 20A), and the product (LAmB-Rh) was complexed with PDD at a weight ratio of 1:10 (LAmB-Rh: PDD). PDD-LAmB and PDD-LAmB-Rh were injected i.p. into MAFIA C57BL/6 mice that express GFP only in macrophages. Control mice received LAmB alone or LAmB complexed with PAMAM-dendrimer coupled to a random untargeted peptide (ScrDD-LAmB). Peritoneal fluid was aspirated one hour after i.p. injection and cells were washed and analyzed by flow cytometry and confocal microscopy. Flow cytometry analysis demonstrated that the percentage of macrophages positive for Rhodamin B was significantly greater in mice treated with PDD-LAmB-Rh than those which received either Rh-LAmB or ScrDR-Rh-LAmB (Fig. 17A). *In vivo* macrophage targeting by PDD/LAmB-Rh (30%+/- 5%) was significantly higher (p<0.001) than that of LAmB-Rh (7.0%+/- 3.7%). Furthermore, macrophages examined with a confocal microscope, using 0.5 micron slices, revealed that within 1 hr, PDD/LAmB-Rh was in the cell (Fig. 17B). The *in vitro* toxicity of LAmB and PDD/LAmB on HEPG2 cells was also extensively analyzed. As shown in the Fig. 17C, PDD significantly reduced LAmB toxicity. PDD reduces LAmB toxicity via dose reduction and reducing off-targeting.

### Efficacy in animal model

PDD-LAmB and the controls described above were then used in a mouse model of therapy for cutaneous *L. major* infection. Female BALB/c mice were inoculated intraperitoneally with 1×10⁶ metacyclic promatigotes of *L. major* (clone VI (MHOM/IL/80/Friedlin). Treatment was started once the lesions reached a surface area between 20 and 70 mm². Mice received intravenous injections of LAmB (37.5 mg/kg/day, the full-dose), LAmB (6.25 mg/kg/day low-dose), PDD-LAmB (6.25 mg/kg/day low-dose with PDD, or 16.7% of full dose), or ScrDR-LAmB (6.25 mg/kg/day or 16.7% of full dose) once a day for 10 consecutive days. Fig. 18A shows that therapy with PDD/LAmB at low-dose was as effective as that of LAmB at full-dose. The skin lesions of the two groups were of comparable size despite different LAmB dosing (Fig. 18A). This finding demonstrates that PDD conjugated with LAmB enhanced the efficacy of LAmB treatment by at least 6 fold (Fig. 18A), which is consistent with increased *in vivo* targeting of macrophages in the peritoneal cavity (Fig. 17A). PDD-LAmB treatment resulted in the closure of skin lesions in 100% of the treated mice (Fig. 18A). The observation that the low-dose LAmB with untargeted nanoparticle (ScrDD-LAmB) treatment has no treatment effect strongly suggests that PDD is required for increased drug efficacy and lowering the required dose of LAmB presumably by targeting LAmB to macrophages. In addition, the kinetics of the closure of skin lesions clearly demonstrated that PDD conjugation accelerated the therapeutic effects of LAmB (17A and Fig. 21). While the full-dose of LAmB required > 12 days to reduce the size of skin lesions, treatment with PDD-LAmB complex containing only 16.7% of the LAmB dose led to a remarkable improvement in skin lesions only after a few days of treatment (Fig. 17A and Fig. 21). Interestingly, real-time PCR amplification and analysis of the parasite burden [17], in the spleen showed that mice treated with PDD-LAmB had a significantly lower (p<0.005) parasite burden than those treated with LAmB (Fig. 18B) representing a ∼86% reduction in the parasite burden.

### PDD-LAmB complex enhanced immune responses to L. major infection

When compared with LAmB treatment, PDD-LAmB therapy resulted in the enlargement of the draining lymph node (Fig. 19A). Moreover, the spleen of PDD-LAmB treated mice was markedly larger than that of mice treated with ScrDR-LAmB (Fig. 19B). Potent immunotherapies or adjuvanted vaccinations are known to result in transient spleen and lymph node enlargement in mice which is associated with a Th1 type response. In addition, MHC II expression in cells isolated from the dorsal area lymph nodes proximal to the skin lesion was statistically greater in mice treated with PDD-LAmB than in mice treated with LAmB and ScrDR-LAmB (Fig. 19C, p < 0.002). Finally, splenocytes of the mice treated with full dose LAmB or those which received low dose LAmB (1/6^{th} of the full dose) complexed either with ScrDR (control nanoparticle), or with PDD were isolated twelve days post treatments. These splenocytes were then co-cultured with a complex composed of HADR and *L. major* gene expressing plasmids (or HADR/empty pVAX) followed by measurement of IFNγ in the supernatants of the splenocytes after 48 hours incubation. By selectively transfecting the APCs in the splenocyte culture, this novel T cell immunomonitoring method allows measurement of antigen-specific T cell responses. This in vitro transfection method uses the complete peptide repertoire of selected *L. major* proteins as the source of antigen, allowing the detection of immune reaction against both known and unknown *L. major* epitopes. Figure 19D shows that PDD-LAmB treatment led to a substantial enhancement of *L. major*-specific IFNγ induction from antigen stimulated splenocytes as compared with full-dose LAmB (p< 0.0001) or control (p< 0.0002).

Following MHC II binding to endogenous peptides, peptide-MHC II complexes are transported to the cell surface, where peptides are presented to CD4⁺ T cells. *Leishmania sp.* inhibits antigen presentation by interfering with the MHC II up-regulation of MHC II molecules. Thus, increased MHC II expression and expansion of CD4⁺ T cells has been explored as a therapeutic approach in the treatment of leishmaniasis. Here, we showed that PDD-mediated enhancement of MHC II expression and CD4⁺ T cell stimulation is associated with elicitation of parasite-specific T cell responses. Indeed, T helper 2 responses, macrophage-mediated immune suppression, and defects in antigen presentation are underlying mechanisms that are used to evade the immune system in leishmaniasis, resulting in increased parasite burden in tissues such as the spleen. The immunoenhancing effects of PDD, in particular in *L. major* infected cells, were shown to induce i) lymphoadenopathy and dendritic cell expansion and trafficking (Fig. 19A,and 19B), ii) enhanced MHC II expression in cells in the draining lymph node (Fig. 19C), which indicates cell activation and increased antigen presentation, iii) and most importantly, the elevation of parasite-specific T cell responses (Fig. 19D).

The data presented here provides evidence that PDD reduces the effective LAmB dose by ∼80% while also showing faster efficacy and lower toxicity. PDD binds LAmB and escorts it selectively to parasite reservoir cells (phagocytes) while its intrinsic CD4⁺ T helper universal peptide provides "HELP" for the host adaptive immunity resulting in dramatic increased *L. major* antigen-specific T cell responses.

### Other Embodiments

Any improvement may be made in part or all of the compositions, kits, platforms, and method steps. The use of any and all examples, or exemplary language (e.g., "such as") provided herein, is intended to illuminate the invention. For example, although the experiments described herein involve the treatment of *Leishmania* infection, the compositions and methods described herein can find use in a number of other therapeutic and prophylactic applications, including inducing an immune response and thus immunity against any antigen of interest (e.g., antigens from infectious pathogens). Additionally, the peptide-derivatized dendrimer (PDD) nanocarrier platform described herein can be complexed with a nucleic acid, a polypeptide or a peptide rather than a drug (e.g., a compound) for treating the disorders and diseases described herein (e.g., *Leishmania* infection).

## Claims

1. A composition for use in therapy, the composition comprising at least one dendrimer having conjugated thereto at least one MHC II targeting moiety and at least one drug, wherein the at least one MHC II targeting moiety is conjugated to the exterior surface of the at least one dendrimer such that the at least one MHC II targeting moiety specifically binds to antigen presenting cells (APCs), and the at least one drug is encapsulated by the at least one dendrimer or conjugated to the exterior surface of the at least one dendrimer, and wherein the at least one drug is any form of a unilamellar liposomal formulation of amphotericin B.

2. The composition for use of claim 1, wherein the at least one drug is encapsulated in a liposome with a net-charge opposite to that of the at least one dendrimer, preferably the charge of the at least one dendrimer is negative and the net-charge of the at least one drug and the liposome is positive or the charge of the at least one dendrimer is positive and the net-charge of the at least one drug and the liposome is negative.

3. The composition for use of claim 1, wherein the at least one MHC II targeting moiety is a peptide.

4. The composition for use of claim 3, wherein the peptide is covalently attached to the at least one dendrimer.

5. The composition for use of claim 1, wherein the at least one drug comprises a liposome.

6. The composition for use of claim 5, wherein the liposome is non-covalently attached to the at least one dendrimer.

7. The composition for use of claim 1, further comprising a pharmaceutically acceptable carrier.

8. The composition for use of claim 1, wherein the at least one MHC II targeting moiety is a Pan- DR T helper epitope (PADRE).

9. The composition for use of claim 1, wherein the at least one dendrimer is a poly-amidoamine (PAMAM) dendrimer.

10. The composition for use of claim 1, wherein the anti-pathogen agent is effective at reducing growth of or killing a pathogen selected from the group consisting of: Leishmania, malaria, tuberculosis, and human immunodeficiency virus (HIV).

11. The composition for use of claim 1, wherein the composition is used in treatment of a subject at risk of contracting or having an intracellular pathogen infection, the use comprising:
administering to the subject an effective amount of the composition of claim 1 to induce MHC class II mediated activation of helper T cells in the subject, and to inhibit growth of or eliminate the intracellular pathogen, wherein administering the composition to the subject results in production of monoclonal antibodies against the intracellular pathogen.

## Patentansprüche

1. Zusammensetzung zur Verwendung in der Therapie, wobei die Zusammensetzung mindestens ein Dendrimer, das daran konjugiert mindestens eine auf MHC II abzielende Einheit aufweist, und mindestens einen Wirkstoff umfasst, wobei die mindestens eine auf MHC II abzielende Einheit an die Außenfläche des mindestens einen Dendrimers konjugiert ist, sodass die mindestens eine auf MHC II abzielende Einheit spezifisch an antigenpräsentierende Zellen (APCs) bindet, und der mindestens eine Wirkstoff durch das mindestens eine Dendrimer eingekapselt oder an die Außenfläche des mindestens einen Dendrimers konjugiert ist, und wobei der mindestens eine Wirkstoff eine Form einer Formulierung von Amphotericin B aus unilamellaren Liposomen ist.

2. Zusammensetzung zur Verwendung nach Anspruch 1, wobei der mindestens eine Wirkstoff in einem Liposom mit einer Nettoladung, die entgegengesetzt der des mindestens einen Dendrimers ist, eingekapselt ist, vorzugsweise ist die Ladung des mindestens einen Dendrimers negativ und ist die Nettoladung des mindestens einen Wirkstoffs und des Liposoms positiv oder ist die Ladung des mindestens einen Dendrimers positiv und ist die Nettoladung des mindestens einen Wirkstoffs und des Liposoms negativ.

3. Zusammensetzung zur Verwendung nach Anspruch 1, wobei die mindestens eine auf MHC II abzielende Einheit ein Peptid ist.

4. Zusammensetzung zur Verwendung nach Anspruch 3, wobei das Peptid kovalent an das mindestens eine Dendrimer gebunden ist.

5. Zusammensetzung zur Verwendung nach Anspruch 1, wobei der mindestens eine Wirkstoff ein Liposom umfasst.

6. Zusammensetzung zur Verwendung nach Anspruch 5, wobei das Liposom nichtkovalent an das mindestens eine Dendrimer gebunden ist.

7. Zusammensetzung zur Verwendung nach Anspruch 1, weiterhin umfassend einen pharmazeutisch akzeptablen Träger.

8. Zusammensetzung zur Verwendung nach Anspruch 1, wobei die mindestens eine auf MHC II abzielende Einheit ein Pan-DR-Epitop (PADRE), ein T-Helfer-Epitop, ist.

9. Zusammensetzung zur Verwendung nach Anspruch 1, wobei das mindestens eine Dendrimer ein Polyamidoamin-Dendrimer (PAMAM-Dendrimer) ist.

10. Zusammensetzung zur Verwendung nach Anspruch 1, wobei das antipathogene Mittel wirksam ist bei der Verringerung des Wachstums oder der Abtötung eines Pathogens, ausgewählt aus der Gruppe bestehend aus: *Leishmania,* Malaria, Tuberkulose und Humanem Immundefizienz-Virus (HIV).

11. Zusammensetzung zur Verwendung nach Anspruch 1, wobei die Zusammensetzung bei der Behandlung eines Individuums mit dem Risiko, an einer Infektion mit einem intrazellulären Pathogen zu erkranken oder eine Infektion mit einem intrazellulären Pathogen aufzuweisen, verwendet wird, wobei die Verwendung umfasst:
Verabreichen an das Individuum einer wirksamen Menge der Zusammensetzung nach Anspruch 1, um eine MHC-Klasse-II-vermittelte Aktivierung von T-Helferzellen bei dem Individuum zu induzieren, und um das Wachstum des intrazellulären Pathogens zu hemmen oder das intrazelluläre Pathogen zu eliminieren, wobei das Verabreichen der Zusammensetzung an das Individuum zur Produktion von monoklonalen Antikörpern gegen das intrazelluläre Pathogen führt.

## Revendications

1. Composition pour utilisation en thérapie, la composition comprenant au moins un dendrimère sur lequel est conjugué au moins un groupement de ciblage CMH II et au moins un médicament, l'au moins un groupement de ciblage CMH II étant conjugué à la surface extérieure de l'au moins un dendrimère de telle sorte que l'au moins un groupement de ciblage CMH II se lie spécifiquement à des cellules présentatrices d'antigènes (CPA), et l'au moins un médicament étant encapsulé par l'au moins un dendrimère ou conjugué à la surface extérieure de l'au moins un dendrimère, et l'au moins un médicament étant une forme quelconque d'une formulation liposomale unilamellaire d'amphotéricine B.

2. Composition selon la revendication 1, l'au moins un médicament étant encapsulé dans un liposome avec une charge nette opposée à celle de l'au moins un dendrimère, la charge de l'au moins un dendrimère étant de préférence négative et la charge nette de l'au moins un médicament et du liposome étant positive ou la charge de l'au moins un dendrimère étant positive et la charge nette de l'au moins un médicament et du liposome étant négative.

3. Composition pour utilisation selon la revendication 1, l'au moins un groupement de ciblage CMH II étant un peptide.

4. Composition pour utilisation selon la revendication 3, le peptide étant lié de façon covalente à l'au moins un dendrimère.

5. Composition pour utilisation selon la revendication 1, l'au moins un médicament comprenant un liposome.

6. Composition pour utilisation selon la revendication 5, le liposome étant lié de façon non covalente à l'au moins un dendrimère.

7. Composition pour utilisation selon la revendication 1, comprenant en outre un support pharmaceutiquement acceptable.

8. Composition pour utilisation selon la revendication 1, l'au moins un groupement de ciblage CMH II étant un épitope T Helper Pan DR (PADRE).

9. Composition pour utilisation selon la revendication 1, l'au moins un dendrimère étant un dendrimère poly(amidoamine) (PAMAM).

10. Composition pour utilisation selon la revendication 1, l'agent anti-pathogène étant efficace pour réduire la croissance de, ou tuer, un pathogène sélectionné parmi le groupe se composant de : *Leishmania,* malaria, tuberculose et virus de l'immunodéficience humaine (VIH).

11. Composition pour utilisation selon la revendication 1, la composition étant utilisée dans le traitement d'un sujet à risque de contracter, ou d'avoir, une infection pathogène intracellulaire, l'utilisation comprenant :
administrer au sujet une quantité efficace de la composition selon la revendication 1 pour entraîner l'activation induite par CMH II de cellules T helper dans le sujet, et pour inhiber la croissance de, ou éliminer, le pathogène intracellulaire, administrer la composition au sujet résultant dans la production d'anticorps monoclonaux contre le pathogène intracellulaire.


### SEQUENCE LISTING

<110> UNIVERSITY OF MIAMI
<120> IMMUNE-POTENTIATING DRUG NANOCARRIERS AND METHODS OF PRODUCTION
   AND USE THEREOF
<130> P13957WO/EP
<140>
   <141>
<150> 61/788,666
   <151> 2013-03-15
<160> 34
<170> PatentIn version 3.5
<210> 1
   <211> 13
   <212> PRT
   <213> Artificial Sequence
<220>
   <223> Description of Artificial Sequence: Synthetic peptide
<220>
   <221> MOD_RES
   <222> (3)..(3)
   <223> Cyclohexylalanine
<400> 1
   Ala Lys Ala Val Ala Ala Trp Thr Leu Lys Ala Ala Ala
   1               5                   10
<210> 2
   <211> 7
   <212> PRT
   <213> Artificial Sequence
<220>
   <223> Description of Artificial Sequence: Synthetic peptide
<400> 2
   Gly Ser Gly Gly Gly Gly Ser
   1               5
<210> 3
   <211> 4
   <212> PRT
   <213> Artificial Sequence
<220>
   <223> Description of Artificial Sequence: Synthetic peptide
<400> 3
   Gly Phe Leu Gly
   1
<210> 4
   <211> 16
   <212> PRT
   <213> Artificial Sequence
<220>
   <223> Description of Artificial Sequence: Synthetic peptide
<220>
   <223> N-term Ac
<220>
   <221> MOD_RES
   <222> (1)..(1)
   <223> D-Alanine
<220>
   <221> MOD_RES
   <222> (3)..(3)
   <223> Cyclohexylalanine
<220>
   <221> MOD_RES
   <222> (14)..(14)
   <223> D-Alanine
<220>
   <221> MOD_RES
   <222> (15)..(15)
   <223> Aminohexanoic acid
<220>
   <223> C-term OH
<400> 4
   Ala Lys Ala Val Ala Ala Trp Thr Leu Lys Ala Ala Ala Ala Xaa Cys
   1               5                   10                  15
<210> 5
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (3)..(3)
   <223> Cyclohexylalanine
<220>
   <221> MOD_RES
   <222> (13)..(13)
   <223> D-Alanine
<400> 5
   Ala Lys Ala Val Ala Ala Trp Thr Leu Lys Ala Ala Ala
   1               5                   10
<210> 6
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 6
   Ala Lys Phe Val Ala Ala Trp Thr Leu Lys Ala Ala Ala
   1               5                   10
<210> 7
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 7
   Ala Lys Tyr Val Ala Ala Trp Thr Leu Lys Ala Ala Ala
   1               5                   10
<210> 8
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 8
   Ala Lys Phe Val Ala Ala Tyr Thr Leu Lys Ala Ala Ala
   1               5                   10
<210> 9
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (3)..(3)
   <223> Cyclohexylalanine
<220>
   <221> MOD_RES
   <222> (13)..(13)
   <223> D-Alanine
<400> 9
   Ala Lys Ala Val Ala Ala Tyr Thr Leu Lys Ala Ala Ala
   1               5                   10
<210> 10
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 10
   Ala Lys Tyr Val Ala Ala Tyr Thr Leu Lys Ala Ala Ala
   1               5                   10
<210> 11
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 11
   Ala Lys Phe Val Ala Ala His Thr Leu Lys Ala Ala Ala
   1               5                   10
<210> 12
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (3)..(3)
   <223> Cyclohexylalanine
<220>
   <221> MOD_RES
   <222> (13)..(13)
   <223> D-Alanine
<400> 12
   Ala Lys Ala Val Ala Ala His Thr Leu Lys Ala Ala Ala
   1               5                   10
<210> 13
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 13
   Ala Lys Tyr Val Ala Ala His Thr Leu Lys Ala Ala Ala
   1               5                   10
<210> 14
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 14
   Ala Lys Phe Val Ala Ala Asn Thr Leu Lys Ala Ala Ala
   1               5                   10
<210> 15
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (3)..(3)
   <223> Cyclohexylalanine
<220>
   <221> MOD_RES
   <222> (13)..(13)
   <223> D-Alanine
<400> 15
   Ala Lys Ala Val Ala Ala Asn Thr Leu Lys Ala Ala Ala
   1               5                   10
<210> 16
   <211> 13
   <212> PRT
   <213> Artificial Sequence
<220>
   <223> Description of Artificial Sequence: Synthetic peptide
<220>
   <221> MOD_RES
   <222> (1)..(1)
   <223> D-Alanine
<220>
   <221> MOD_RES
   <222> (13)..(13)
   <223> D-Alanine
<400> 16
   Ala Lys Tyr Val Ala Ala Asn Thr Leu Lys Ala Ala Ala
   1               5                   10
<210> 17
   <211> 13
   <212> PRT
   <213> Artificial Sequence
<220>
   <223> Description of Artificial Sequence: Synthetic peptide
<220>
   <221> MOD_RES
   <222> (3)..(3)
   <223> Cyclohexylalanine
<400> 17
   Ala Lys Ala Val Ala Ala Trp Thr Leu Lys Ala Ala Ala
   1               5                   10
<210> 18
   <211> 13
   <212> PRT
   <213> Artificial Sequence
<220>
   <223> Description of Artificial Sequence: Synthetic peptide
<400> 18
   Ala Lys Phe Val Ala Ala Trp Thr Leu Lys Ala Ala Ala
   1               5                   10
<210> 19
   <211> 13
   <212> PRT
   <213> Artificial Sequence
<220>
   <223> Description of Artificial Sequence: Synthetic peptide
<400> 19
   Ala Lys Tyr Val Ala Ala Trp Thr Leu Lys Ala Ala Ala
   1               5                   10
<210> 20
   <211> 13
   <212> PRT
   <213> Artificial Sequence
<220>
   <223> Description of Artificial Sequence: Synthetic peptide
<400> 20
   Ala Lys Phe Val Ala Ala Tyr Thr Leu Lys Ala Ala Ala
   1               5                   10
<210> 21
   <211> 13
   <212> PRT
   <213> Artificial Sequence
<220>
   <223> Description of Artificial Sequence: Synthetic peptide
<220>
   <221> MOD_RES
   <222> (3)..(3)
   <223> Cyclohexylalanine
<400> 21
   Ala Lys Ala Val Ala Ala Tyr Thr Leu Lys Ala Ala Ala
   1               5                   10
<210> 22
   <211> 13
   <212> PRT
   <213> Artificial Sequence
<220>
   <223> Description of Artificial Sequence: Synthetic peptide
<400> 22
   Ala Lys Tyr Val Ala Ala Tyr Thr Leu Lys Ala Ala Ala
   1 5 10
<210> 23
   <211> 13
   <212> PRT
   <213> Artificial Sequence
<220>
   <223> Description of Artificial Sequence: Synthetic peptide
<400> 23
   Ala Lys Phe Val Ala Ala His Thr Leu Lys Ala Ala Ala
   1 5 10
<210> 24
   <211> 13
   <212> PRT
   <213> Artificial Sequence
<220>
   <223> Description of Artificial Sequence: Synthetic peptide
<220>
   <221> MOD_RES
   <222> (3)..(3)
   <223> Cyclohexylalanine
<400> 24
   Ala Lys Ala Val Ala Ala His Thr Leu Lys Ala Ala Ala
   1 5 10
<210> 25
   <211> 13
   <212> PRT
   <213> Artificial Sequence
<220>
   <223> Description of Artificial Sequence: Synthetic peptide
<400> 25
   Ala Lys Tyr Val Ala Ala His Thr Leu Lys Ala Ala Ala
   1 5 10
<210> 26
   <211> 13
   <212> PRT
   <213> Artificial Sequence
<220>
   <223> Description of Artificial Sequence: Synthetic peptide
<400> 26
   Ala Lys Phe Val Ala Ala Asn Thr Leu Lys Ala Ala Ala
   1               5                   10
<210> 27
   <211> 13
   <212> PRT
   <213> Artificial Sequence
<220>
   <223> Description of Artificial Sequence: Synthetic peptide
<220>
   <221> MOD_RES
   <222> (3)..(3)
   <223> Cyclohexylalanine
<400> 27
   Ala Lys Ala Val Ala Ala Asn Thr Leu Lys Ala Ala Ala
   1               5                   10
<210> 28
   <211> 13
   <212> PRT
   <213> Artificial Sequence
<220>
   <223> Description of Artificial Sequence: Synthetic peptide
<400> 28
   Ala Lys Tyr Val Ala Ala Asn Thr Leu Lys Ala Ala Ala
   1               5                   10
<210> 29
   <211> 21
   <212> PRT
   <213> Artificial Sequence
<220>
   <223> Description of Artificial Sequence: Synthetic peptide
<400> 29
   Phe Asn Asn Phe Thr Val Ser Phe Trp Leu Arg Val Pro Lys Val Ser
   1               5                   10                  15
   Ala Ser His Leu Glu
               20
<210> 30
   <211> 15
   <212> PRT
   <213> Artificial Sequence
<220>
   <223> Description of Artificial Sequence: Synthetic peptide
<400> 30
   Ser Ser Val Phe Asn Val Val Asn Ser Ser Ile Gly Leu Ile Met
   1               5                   10                  15
<210> 31
   <211> 12
   <212> PRT
   <213> Artificial Sequence
<220>
   <223> Description of Artificial Sequence: Synthetic peptide
<400> 31
   Ser Lys Met Arg Met Ala Thr Pro Leu Leu Met Gln
   1               5                   10
<210> 32
   <211> 15
   <212> PRT
   <213> Artificial Sequence
<220>
   <223> Description of Artificial Sequence: Synthetic peptide
<400> 32
   Gln Tyr Ile Lys Ala Asn Ser Lys Phe Ile Gly Ile Thr Glu Leu
   1               5                   10                  15
<210> 33
   <211> 15
   <212> PRT
   <213> Artificial Sequence
<220>
   <223> Description of Artificial Sequence: Synthetic peptide
<220>
   <221> MOD_RES
   <222> (1)..(1)
   <223> D-Alanine or Alanine
<220>
   <221> MOD_RES
   <222> (3)..(3)
   <223> Cyclohexylalanine
<220>
   <221> MOD_RES
   <222> (13)..(13)
   <223> D-Alanine or Alanine
<220>
   <221> MOD_RES
   <222> (14)..(14)
   <223> Aminocaproic acid
<400> 33
   Ala Lys Ala Val Ala Ala Trp Thr Leu Lys Ala Ala Ala Xaa Cys
   1               5                   10                  15
<210> 34
   <211> 12
   <212> PRT
   <213> Artificial Sequence
<220>
   <223> Description of Artificial Sequence: Synthetic peptide
<400> 34
   Ser Phe Glu Arg Phe Glu Ile Phe Pro Lys Glu Cys
   1               5                   10